# EUROPEAN PATENT SPECIFICATION

(11) **EP 2 872 621 B1**
(45) Date of publication and mention of the grant of the patent: **31.10.2018**
(21) Application number: 13739653.7
(22) Date of filing: 16.07.2013
(51) Int. Cl.: C12N 5/0786, A61K 35/14, A61P 37/00

(54) **METHOD FOR THE GENERATION OF NON-TRANSFORMED MACROPHAGE CELL LINES**
VERFAHREN ZUR ERZEUGUNG NICHT TRANSFORMIERTER MAKROPHAGENZELLLINIEN
PROCÉDÉ POUR LA GÉNÉRATION DE LIGNÉES CELLULAIRES DE MACROPHAGES NON TRANSFORMÉS

(30) Priority: 16.07.2012 EP 12176565
(43) Date of publication of application: 20.05.2015
(73) Proprietor: Max-Planck-Gesellschaft zur Förderung der Wissenschaften e.V., 80539 München (DE)
(72) Inventor: FEJER, György, Saltash Cornwall PL12 6DP (GB); FREUDENBERG, Marina, 79194 Gundelfingen (DE); GYÓRY, Ildikó, Saltash Cornwall PL12 6DP (GB); GALANOS, Chris, 79194 Gundelfingen (DE)
(74) Representative: Vossius & Partner Patentanwälte Rechtsanwälte mbB
(86) International application number: PCT/EP2013/065015
(87) International publication number: WO 2014/012933

(56) References cited:
- WO-A1-94/26875
- WO-A1-99/16455
- DENHAM S ET AL: "Granulocyte-macrophage colony stimulating factor promotes prolonged survival and the support of virulent infection by African swine fever virus of macrophages generated from porcine bone marrow and blood.", THE JOURNAL OF GENERAL VIROLOGY, vol. 77 ( Pt 10), October 1996 (1996-10), pages 2625-2630, XP002688768, ISSN: 0022-1317 cited in the application
- ESKE K ET AL: "Generation of murine bone marrow derived macrophages in a standardised serum-free cell culture system.", JOURNAL OF IMMUNOLOGICAL METHODS, vol. 342, no. 1-2, 15 March 2009 (2009-03-15), pages 13-19, XP002688769, ISSN: 1872-7905
- LANDSMAN L & JUNG S: "Lung macrophages serve as obligatory intermediate between blood monocytes and alveolar macrophages.", JOURNAL OF IMMUNOLOGY, vol. 179, no. 6, 15 September 2007 (2007-09-15), pages 3488-3494, XP002688770, ISSN: 0022-1767 cited in the application
- SHIBATA Y ET AL: "GM-CSF regulates alveolar macrophage differentiation and innate immunity in the lung through PU.1.", IMMUNITY, vol. 15, no. 4, October 2001 (2001-10), pages 557-567, XP002688771, ISSN: 1074-7613 cited in the application

## Description

The present invention relates to a method for producing self-renewing, non-transformed macrophages comprising: culturing a cell preparation obtained from an organ obtained from a mammal in culture medium to which granulocyte macrophage colony-stimulating factor (GM-CSF) has been added; thereby differentiating the cell population into self-renewing, non-transformed macrophages, wherein the self-renewing, non-transformed macrophages are characterised by the expression of at least four markers selected from the group consisting of GM-CSF receptor, M-CSF receptor, CD11c, TLR2, TLR4, TLR3, F4/80, CD16, CD11b, CD14 and Marco, and are characterised by a lack of expression of at least 3 markers selected from the group consisting of B220, Gr1, IgE receptor, T-cell surface glycoprotein CD3 epsilon chain and B-cell receptor. The present invention further relates to macrophages obtainable by the method of the invention, wherein the macrophages are self-renewing, non-transformed macrophages being characterised by the expression of at least four markers selected from the group consisting of GM-CSF receptor, M-CSF receptor, CD11c, TLR2, TLR4, TLR3, F4/80, CD16, CD11b, CD14 and Marco, and being characterised by a lack of expression of at least 3 markers selected from the group consisting of B220, Gr1, IgE receptor, T-cell surface glycoprotein CD3 epsilon chain and B-cell receptor as well as their use in medicine or medical/pharmaceutical research. Furthermore, the present invention relates to a method of identifying a compound having a pharmacological, differentiating, proliferative or anti-proliferative, cytotoxic or transforming effect on the self-renewing, non-transformed macrophages of the invention as well as a method of identifying a compound capable of treating a disease selected from the group consisting of an inflammatory disease, infections, asthma, contact allergies as well as the side effects associated with virus vector gene-therapy.

Macrophages comprise a heterogeneous population of tissue resident mononuclear phagocytes, crucially involved in anti-microbial defence and steady-state tissue homeostasis (Geissmann et al., 2010a; Geissmann et al., 2010b). A major part of them originates from blood monocytes of bone-marrow (BM) progenitors mainly under the influence of M-CSF signalling (Fogg et al., 2006; Gow et al., 2010). Macrophages colonize various organs where further cellular differentiation occurs resulting in specialized macrophages with a limited life-span (Geissmann et al., 2010b). However, some subsets of mononuclear phagocytes derive from embryonic yolk-sac but not from BM hematopoetic stem cells and persist in different tissues in the adult mouse (Schulz et al., 2012). Furthermore, microglia (Ajami et al., 2007; Ginhoux et al., 2010), Langerhans cells (Chorro et al., 2009; Merad et al., 2002), alveolar macrophages (Hume et al., 2002; Landsman and Jung, 2007) and pleural macrophages (Jenkins et al., 2011) have been shown to proliferate independently of the BM. Nevertheless, the developmental origin of all tissue macrophage subsets is still not completely understood.

Based on their responses to stimuli and their naïve gene expression patterns, macrophages show two major types of activation: classically activated or M1 macrophages being proinflammatory and promoting Th1 responses and alternatively activated or M2-macrophages involved in tissue remodelling and supporting Th2 effector functions (Biswas and Mantovani, 2010; Gordon and Martinez, 2010). Whether this division completely reflects macrophage plasticity *in vitro* and *in vivo* is still unclear at present.

Pathogen sensing and the production of pro- and anti-inflammatory mediators are important functions of macrophages. Pathogen sensing is mediated by a number of pattern recognition receptors encompassing scavenger receptors, C-type lectins, TLRs, NLRs and RLRs (Kawai and Akira, 2011). Both quantitative and qualitative differences in receptor distribution and cytokine production have been reported among distinct macrophage populations and the role of various pattern recognition receptors and signalling pathways in macrophage activation has been under intensive investigation. Usually, primary macrophages isolated from tissues, ex *vivo* differentiated cells and immortalized macrophage lines are being utilized.
However, the use of freshly made primary cells is hampered by the elaborate isolation procedures, purity, limited cell numbers and the large number of human or animal donors required.
Macrophage lines such as e.g. human and murine lines have been described that were obtained after spontaneous or experimentally-induced transformation by oncogenes. Such cells, however, differ from the primary cells due to this transformation.
Because of the drawbacks associated with primary cells and transformed cell lines, macrophages obtained by culturing bone marrow cells in M-CSF containing medium (BMM) are often employed for functional studies and high-throughput screening. However, these cells have a limited lifespan in culture and, moreover, delineate only one particular set of macrophage populations (Chow et al., 2011; Geissmann et al., 2010a).

Talbot and Paape (1996) describe a continuous culture of non-transformed macrophages derived from fetal or newborn pig testicle and liver explants. The cells were mechanically separated from the explants and subsequently cultured on feeder layers of STO mouse embryonic fibroblasts in 10% DMEM-199. The presence of feeder cells was shown to be required, as it inhibits the outgrowth of contaminating fibroblasts in the culture. The authors found that liver-derived cultures could be maintained for 15 to 18 passages before proliferation stopped, while testicle-derived cultures could be maintained at a relatively low population density to beyond passage 30. More importantly, however, the onset of senescence started at about the 10th passage in both types of cultures, which significantly reduced the number of macrophages that could be harvested.

Denham *et al.* (1996) describe cultures of non-adherent cells of the monocyte-macrophage lineage derived from the bone marrow and blood of weanling pigs by culturing these cells in the presence of recombinant porcine granulocyte-macrophage colony stimulating factor (GM-CSF). The cells obtained maintain their potential to be infected with the African swine fever virus (ASFV). The authors detail in the results that the cells obtained were non-adherent and had a dendritic appearance. Furthermore, the cells stained positive for an antigen specific for porcine granulocytes and macrophages, putative CD11b, CD14 and CD44 and were negative for CD1, CD4 and CD8. Approximately 50% of the cells further stained positive for MHC class II DQ and DR. Although the authors point out that the cells could be maintained in culture for three months or longer, they also show that the initially observed increase in cell numbers slowed to low levels beyond four weeks.

Inaba *et al*. (1992) describe the use of GM-CSF in the generation of large numbers of dendritic cells from mouse bone marrow cultures. The dendritic cells thus obtained are characterised as being MHC class II-positive and are non-adherent cells. The authors further describe that the use of M-CSF instead of GM-CSF resulted in the growth of large numbers of macrophages instead of dendritic cells, while the application of a mixtures of both GM-CSF and M-CSF resulted in mixed cultures.

Lutz *et al*. (1999) describe an improved method for the generation of a highly pure population of dendritic cells from a prolonged culture of bone marrow in the presence of GM-CSF. This method represents a further development of the above described method described by Inaba *et al*. (1992) and, according to the authors, using GM-CSF as the sole cytokine results in 90 to 95% pure populations of immature and mature bone marrow-derived dendritic cells with a limited life-span. The rest of the cells do not continue to proliferate and no potential macrophage like functions (phagocytosis, cytokine production to external stimulation) have been shown for said cells.

Thus, despite the fact that a lot of effort has been invested into methods to establish macrophage cell cultures, no method exists so far that enable the long-term culture of macrophages without changing the properties of these cells, i.e. without the need for artificial modifications of the cells such as cell transformation with viral oncogenes or rendering the cells tumorigenic. Accordingly, there is still a need to provide such methods and such cell cultures, for example for disease modelling and drug discovery.

This need is addressed by the provision of the embodiments characterised in the claims.

Accordingly, the present invention relates to a method for producing self-renewing, non-transformed macrophages comprising: culturing a cell preparation obtained from an organ obtained from a mammal in culture medium to which granulocyte macrophage colony-stimulating factor (GM-CSF) has been added; thereby differentiating the cell population into self-renewing, non-transformed macrophages, wherein the self-renewing, non-transformed macrophages are characterised by the expression of at least four markers selected from the group consisting of GM-CSF receptor, M-CSF receptor, CD11c, TLR2, TLR4, TLR3, F4/80, CD16, CD11b, CD14 and Marco, and are characterised by a lack of expression of at least 3 markers selected from the group consisting of B220, Gr1, IgE receptor, T-cell surface glycoprotein CD3 epsilon chain and B-cell receptor.

The self-renewing macrophages of the present invention are also referred to herein as MPI cells.

The term "self-renewing", as used herein, is defined in accordance with the pertinent art and relates to the ability of cells to go through numerous cycles of cell division. Preferably, the self-renewing macrophages of the present invention maintain their ability to divide for at least 12 passages, such as for example at least 15 passages, such as for example at least 20 passages and more preferably at least 25 passages. More preferably, the self-renewing macrophages of the present invention maintain their ability to divide for at least 30 passages, such as for example at least 40 passages, such as for example at least 50 passages, even more preferably at least 75 passages and most preferably at least 100 passages. Most preferably, the self-renewing macrophages of the present invention maintain their ability to divide for an unlimited amount of time.

In accordance with the present invention, the rate of cell division of the cells does not or does not substantially decrease upon continued division as defined above. Accordingly, the rate of proliferation after e.g. 12 or more passages remains substantially the same or even increases as compared to the rate of proliferation observed directly after the cells have been obtained in accordance with the method of the invention (i.e. the initial rate of proliferation). The rate of proliferation is considered to have not substantially decreased as compared to the initial rate of proliferation if it is at least 70% of the initial rate of proliferation, such as e.g. at least 80% of the initial rate of proliferation, more preferably at least 90% of the initial rate of proliferation, such as e.g. at least 95% of the initial rate of proliferation. More preferably, the rate of proliferation is identical to the initial rate of proliferation, and more preferably the rate of proliferation is higher than the initial rate of proliferation, such as e.g. 120% of the initial rate of proliferation, more preferably 150% of the initial rate of proliferation. The skilled person is well aware of how to determine the rate of proliferation. Non-limiting example include determination of the frequency of splitting of the cells required or of cell numbers after a certain period of growth, such as e.g. 24 hours after splitting etc. (Pollard JW., Basic cell culture. Methods Mol Biol. 1990; 5:1-12.).

In addition, it will be appreciated by the skilled person that the self-renewing macrophages of the present invention maintain or essentially maintain their characteristics during self-renewal, even after prolonged periods of time. Such characteristics include, without being limiting, their biological function or their specific marker expression profile.

Non-limiting examples of the biological functions of the self-renewing macrophages of the present invention include their function as phagocytes, the ability for killing of microorganisms, the ability to produce inflammatory mediators such as cytokines, chemokines and interferon and to up-regulate the expression of surface markers (CD69, CD14) important in the activation of innate immune cells in response to stimulation. In addition, their specific marker expression profile includes the presence of at least four markers selected from GM-CSF receptor, M-CSF receptor, CD11c, TLRs, F4/80, CD16, CD11b, CD14 and Marco, and/or a lack of expression of at least three markers selected from B220, Gr1, IgE receptor, T-cell receptor and B-cell receptor, as defined herein below.

The skilled person is aware of how to determine these characteristics. For example, the function of the cells as phagocytes may be determined via methods well known in the art, such as e.g. visualization of internalized microbial particles by microscopy or fluorescent-activated cell sorting (FACS), as described in e.g. in Langermans *et al*. 1994, Eske *et al*. 2009 or Lutz *et al.* 1994. Moreover, methods for determining the ability for killing of microorganisms have been described elsewhere and are fully applicable in accordance with the present invention (see e.g. Descamps *et al*. 2012). Methods for determining the ability to produce inflammatory mediators such as cytokines, chemokines and interferon have also been described in the art and are fully applicable in accordance with the present invention, see e.g. Fejer G, Drechsel L, Liese J, Schleicher U, Ruzsics Z, et al. (2008) Key Role of Splenic Myeloid DCs in the IFN-αβ Response to Adenoviruses In Vivo. PLoS Pathog 4(11): e1000208. doi: 10.1371/journal.ppat.1000208.

Moreover, the expression of specific markers can be determined on the amino acid level as well as on the nucleic acid level by methods well known in the art and described herein below.

In accordance with the present invention, the self-renewing macrophages of the present invention are considered to essentially maintain their characteristics during self-renewal if the degree of similarity between the cells originally obtained by the method of the invention and the cells after self-renewal, such as e.g. after 12 or more passages, is at least 70%, more preferably at least 80%, such as e.g. at least 90% and more preferably at least 95%. Even more preferably, the degree of similarity is at least 99%. The degree of similarity can be determined based on the above described characteristics. For example, a complete expression profile of the self-renewing macrophages after passaging may be compared to the expression profile of originally obtained self-renewing macrophages and the degree of similarity may be determined.

The term "non-transformed", as used herein, relates to the lack of genetic alteration of the cells during the method of the present invention of producing self-renewing macrophages by introduction of exogenous genetic material, such as e.g. viral oncogenes commonly employed in the art, into the cells.

The term "macrophages", as used herein, is defined in accordance with the pertinent art and relates to cells that are phagocytes, acting in both innate immunity as well as cell-mediated immunity of vertebrate animals. Their role is to phagocytose cellular debris and pathogens either as stationary or mobile cells, and to stimulate lymphocytes and other immune cells to respond to the pathogen. Under non-inflammatory conditions macrophages are a heterogeneous population of tissue-resident cells of embryonal liver, bone marrow or blood monocyte origin. During inflammation, new macrophages are recruited from monocytes (Plüddemann A. et al. 2011). Macrophages are generally characterised by the expression of CD14, CD11b or F4/80 (mice)/EMR1 (human) and biological capabilities such as phagocytosis, cytokine and chemokine production etc as described above.

The term "cell preparation", as used herein, relates to a mixtures of cells obtained from a tissue. The cell preparation may be a whole-cell preparation or a preparation of cells in which an enrichment for one or more particular cell types or the removal of certain unwanted cells has been carried out. Preferably, the cell preparation is a whole-cell preparation, i.e. a cell preparation obtained from tissue, wherein all the cells present in said tissue are included in the cell preparation. Accordingly, a cell separation or pre-selection is not carried out in accordance with this preferred embodiment of the method of the present invention.

Whole-cell preparations may be obtained by any method known in the art, such as e.g. described in Seglen 1979 or Locke *et al*. 2009 or as described in the appended examples.

For example, the organ or the part of an organ (both of which are also referred to herein as "the tissue") can be disrupted mechanically, such as e.g. by pipetting the tissue up and down a pipette tip; or by pushing the tissue against a filter or a microstrainer, of for example 10 to 100 µm pore size; or by shaking the tissue in buffer for an appropriate period of time, such as e.g. 10 minutes to 2 hours; or by using e.g. a razor blade or a scalpel to mince the tissue into small pieces; or by a mixture of such methods. Alternatively, or additionally, the tissue may be separated enzymatically using well known enzymes such as for example trypsin, dispase, collagenase or elastase. Tissue disruption may be carried out at temperatures between -5 °C to 40 °C, preferably between 22 °C to 37 °C and most preferably at 37 °C. It is preferred that tissue obtained from liver or spleen is disrupted by mechanical disruption either by pipetting up and down a pipette tip or by pushing the tissue against a filter or is disrupted enzymatically. When employing lung, it is preferred that the tissue is disrupted by mincing the tissue into small pieces, followed by gently shaking in buffer, preferably an isotonic buffer such as e.g. PBS, pH 7.2 for e.g. 1 to 2 hours. The use of enzymatic digestion is less preferred for lung tissue in order to reduce the number of contaminating cells.

In accordance with the method of the present invention, both single-cell suspensions as well as cell preparations comprising or consisting of tissue fragments may be employed. Where tissue fragments are employed, it is preferred that the tissue fragments are fragments consisting of less than 5.000 cells/fragment, such as e.g. less than 2.500 cells/fragment, more preferably less than 1.000 cells/fragment, such as e.g. less than 500 cells/fragment, even more preferably less than 250 cells/fragment, such as e.g. less than 100 cells/fragment and most preferably less than 10 cells per fragment. Where single cell suspensions are to be employed, i.e. where tissue fragments are to be removed, the cells can for example be passed through a filter or a microstrainer; or the cells can be subjected to sedimentation at e.g. 1G; or the cells may be separated by use of magnetic beads that are coupled to one or more macrophage-specific antibodies, such as e.g. anti-CD11b, anti-F4/80 etc., or by density gradients, such as e.g. a Percoll gradient, a Ficol gradient, a Metrizamide gradient etc..

The tissue from which the cell preparation is obtained is an organ or is part of an organ of a mammal. It will be appreciated that where an organ is pivotal for survival of the subject, in particular in humans, only parts of the organ are to be obtained for use in the method of the invention by methods that do not result in a loss of function of said organ. Preferably, at least 50% of the function of said organ is to be maintained, more preferably at least 60%, such as at least 70%, more preferably at least 80%, such as at least 90% and even more preferably at least 95%, such as e.g. at least 98%. Even more preferably at least 99% of the function of the organ is to be maintained and most preferably, the function of the organ is maintained to 100%.

Any organ or part of an organ obtained from a mammal may be employed. Preferably, the organ (or part thereof) is selected from liver, lung, spleen or brain, more preferably the organ is selected from liver, lung or spleen. It will be appreciated that bone marrow or blood does not fall under the term "organ". Accordingly, the cell preparation is not obtained from blood or bone marrow.
Organs from which the cell preparation can be obtained include fetal, neonatal or adult organs. It will be appreciated that where the embryonic, fetal or neonatal organ (or part thereof) is obtained from a human subject, the human embryo, fetus or neonate is not destroyed by taking this organ/part of the organ and the organ or part thereof is solely taken for means of therapy of said embryo, fetus or neonatal human subject.
The term "embryonic organ", as used herein refers to an organ obtained during the first stage of development after conception, whereas the term "fetal organ", as used herein, refers to the later stages of development, preferably the second half of development before birth. Neonatal organs are organs derived from a mammal in the initial period of life after birth. In humans and other large mammals such as pigs and cows, neonatal organs are organs derived during e.g. the first eight weeks after birth. preferably the first four weeks after birth; in rodents, in particular in mice, neonatal organs are organs derived during e.g. the first four weeks after birth, preferably the first two weeks after birth. The term "adult organ" is used herein for organs obtained any time after the end of the neonatal time, i.e. in humans from the beginning of the fifth week of live onwards.

When employing liver as the organ from which the cell preparation is obtained, it is preferred that the liver is a fetal liver or from a newborn subject, i.e. a neonatal liver. Moreover, for spleen, neonatal to adult organs are preferred, while for lung fetal, neonatal as well as adult are preferred.

Means and methods of obtaining tissue are well known in the art. For example, organs or parts of organs may be obtained from a deceased subject or from a living subject, for example during an operation or in form of a biopsy (Henle and Deinhardt 1957; Katsuta *et al.* 1980; Kono *et al*. 1995).

Mammals in accordance with the present invention include all mammals, in particular domestic and farm animals, experimental animals as well as wild animals.

The method of the invention is carried out under suitable cell culture conditions. General cell culture conditions as well as suitable cell culture media are well known in the art (e.g. Cooper GM (2000). "Tools of Cell Biology", ISBN 0-87893-106-6; K. Turksen, ed., Humana Press, 2004, J. Masters, ed., Oxford University Press, 2000, "Animal cell culture", ISBN-10 0-19-963796-2). Preferably, the cell culture conditions comprise conditions of about 2 to 10% CO₂, preferably about 5% CO₂ and a temperature of about 32 to 38°C, preferably about 37°C. Preferably, the cell culture is carried out under sterile conditions. The cells may be cultured for e.g. about one day, two days, three days, four days, five days, six days, seven days, eight days, nine days, ten days, eleven days, twelve days, 13 days, 14 days, 15 days, 16, days, 17 days, 18 days, 19 days, 20 days or most preferably for about 21 days in accordance with the method of the invention.

In accordance with the present invention, any medium suitable as a culture medium for macrophages may be employed. Such media are well established in the art and have been described, e.g. in Pollard 1990 and Weischenfeldt and Porse 2008. For example, the culture medium can be a medium selected from the group consisting of RPMI 1640, Iscove's, F12, OPTI-MEM, DMEM, etc.. Preferably, the medium is supplemented with serum, including non-human or human serum. Preferred amounts of serum to be employed are between 1% and 30%, more preferably between 2 and 20%, such as e.g. between 5 and 15% and most preferably the amount is about 10%. Serum such as for example FCS may be obtained from e.g. GIBCO, Sigma or PAA.

In accordance with the method of the invention, the medium comprises GM-CSF.

GM-CSF is defined in accordance with the pertinent art and relates to granulocyte-macrophage colony-stimulating factor, a cytokine that acts as a growth factor for leukocytes. Human GM-CSF is represented by the GenBank data base entry AAA52578.1 (publication date November 8, 1994), whereas murine GM-CSF is represented by the GenBank data base entry AAB20031.1 (publication date October 11, 2002) and rat GM-CSF is represented by the GenBank data base entry EDM04426.1 (publication date June 20, 2007). GM-CSF has been described in the art, for example in Barreda *et al.* 2004.

Preferred amounts of GM-CSF to be employed are between 0.2 and 20 ng/ml, more preferably between 0.5 and 10 ng/ml, such as e.g. between 1 and 5 ng/ml and most preferably the amount is 2 ng/ml. GM-CSF may be obtained from e.g. R&D Systems, Sigma, Peprotech. In addition, conditioned medium from cell lines producing GM-CSF either spontaneously or after transfection with a respective expression vector, may also be employed.
Preferably, the GM-CSF is recombinant GM-CSF. Moreover, it will be appreciated that it is preferable to employ GM-CSF of the same animal as the cells to be cultured, i.e. where murine cells are to be cultured, the GM-CSF is preferably murine GM-CSF and where human cells are to be cultured, the GM-CSF is preferably human GM-CSF.

Further substances that may optionally be added to the culture medium include, without being limiting, antibiotics, such as e.g. penicillin/streptomycin or amino acid supplements. Preferably, the method of the present invention is carried out in RPMI1640 medium supplemented with 10% FCS, 2ng/ml GM-CSF and penicillin/streptomycin. Preferred amounts of antibiotics are in the range of 50 to 100 units of penicillin and 50 to 100 µg/ml of streptomycin. More preferably, the method of the present invention is carried out in RPMI1640 medium supplemented with solely 10% FCS and 2ng/ml GM-CSF.
It is further preferred that GM-CSF is the only cytokine that is added to the cell culture medium employed in the method of the present invention, whereas cytokines naturally secreted by the cells in the cell culture may be present in said culture. In other words, it is preferred that no cytokines other than GM-CSF are added to the cell culture medium by the experimenter/researcher/medical staff.

It is preferred in any of the cell culture conditions described herein that the medium is exchanged (i.e. refreshed) or refreshed with nutrients and additives, such as e.g. GM-CSF at appropriate intervals, such as e.g. every 24 hours, preferably every two days, more preferably every three days, such as e.g. every four days, once a week or every two weeks. Most preferably, the medium is exchanged for fresh cell culture medium once a week. When factors are added to the medium instead of exchanging medium, then it will be appreciated that the medium is nonetheless exchanged for fresh medium in regular intervals, such as at least once every two weeks, more preferably once a week.
Moreover, the skilled person is aware that upon culturing the cells under appropriate conditions, high density or confluence of the cells occurs, such that the cells may need to be split (e.g. passaged). After splitting, the cells can be re-plated at a diluted concentration into e.g. an increased number of culture dishes, flaks or onto solid supports. With increasing passage number, the amount of cells obtained can therefore be increased due to cell division. Where an increase in cell numbers is not required/wanted, a portion of the cells may be re-plated at a diluted concentration and the remainder of the cells be discarded.
The skilled person is aware of means and methods for splitting cells and can determine the appropriate time point and dilution for splitting cells. For example, cells may be removed from their culture vessel by addition of 1.5mM EDTA/PBS and diluted in fresh culture medium. Preferably, cells are split between 1:3 and 1:10, preferably 1:5 when the cells have reached confluence, such as e.g. every three, every four days, every five days, every six days, every seven days, every eight days, every nine days or every ten days. Preferably, cells are split every five to eight days.

Particularly suitable culture conditions and media are shown in detail in the examples of the invention.

Means and methods of obtaining cell cultures derived from a single cell, if required, are well known in the art. For example, the cells obtained by the method of the invention can be subjected to limited dilution as described e.g. in Wen *et al*. 1987 or cell sorting approaches, such as e.g. magnetic activated cell sorting (MACS) or flow cytometry activated cell sorting (FACS) or panning approaches using immobilised antibodies as described for example in Dainiak et al. (Adv Biochem Eng Biotechnol. 2007;106:1-18). Suitable markers for use in these methods include the characterising markers of the self-renewing macrophages of the invention as described elsewhere herein.

Innate immune responses triggered by microbial components in macrophages are of eminent importance for combating infection but at the same time they can provoke inflammatory disorders. Usually, freshly isolated macrophages from different tissues or macrophages generated from bone marrow are used to study the microbe-induced responses of mouse primary cells. The relatively small number of tissue macrophages obtained and the lack of methods to keep non-transformed tissue- and bone marrow-derive macrophages to proliferate *in vitro* complicate such investigations. In accordance with the present invention, it was now surprisingly found that a macrophage cell line can be generated from mixed cell preparations from different mammalian organs. As is shown in the appended examples, cultivation of such mixed whole-cell preparations in the presence of GM-CSF resulted in the generation of self-renewing cells expressing macrophage markers. So far, these cells exhibit an unlimited life span (3 years at present) without changing their properties. Transformation of the cells, as is commonly employed in the art in order to immortalise cells, is not required in order to generate these self-renewing macrophages. However, the presence of GM-CSF is required in order to maintain the cells proliferating. Accordingly, the cells obtained are also referred to herein as "GM-CSF-dependent macrophages". To the inventors' best knowledge, there are no described examples in the available literature that macrophages with a virtually unlimited life span can be obtained after cell culture with GM-CSF as the sole cytokine factor in the medium. This method offers a simple, less cost-intensive and very well reproducible method to obtain differentiated macrophages. Because these cells are non-transformed, the possibility of losing some of the characteristics of the immune reactivity is minimal. In addition, the cells of the present invention are non-tumorigenic when administered to animals *in vivo,* as shown in Example 2.
In several aspects, including morphological appearance of surface marker expression patterns, requirements for sensing bacterial lipopolysaccharide (LPS), high sensitivity to adenovirus (Ad) infection and a unique, highly pro-inflammatory pattern of cytokine response to microbial agents, the cells of the present invention are very similar to alveolar macrophages (AMs). Thus the cells of the present invention represent a new valuable tool for studies into the properties and functions of tissue macrophages.

In a preferred embodiment, the cells are cultured for at least about 21 days. More preferably, the cells are cultured for at least about 28 days, even more preferably for at least about 56 days, and most preferably for at least about 84 days.

The term "at least", as used herein, refers to the specifically recited amount or number but also to more than the specifically recited amount or number. For example, the term "at least 21 days" encompasses also at least 22, at least 23, at least 24, at least 25, at least 26, at least 27, at least 28, at least 29 days, such as at least 30, at least 40, at least 50, at least 60, at least 70, at least 100 days, at least 150 days, at least 200 days, at least 300 days, at least 400 days, at least 500 days, at least 600 days, at least 700 days, at least 730 days, at least 800 days, at least 900 days and so on. Furthermore, this term also encompasses exactly 21, exactly 22, exactly 23, exactly 24, exactly 25, exactly 26, exactly 27, exactly 28, exactly 29, exactly 30, exactly 40, exactly 50, exactly 60, exactly 70, exactly 100 days, exactly 150 days, exactly 200 days, exactly 300 days, exactly 400 days, exactly 500 days, exactly 600 days, exactly 700 days, exactly 730 days, exactly 800 days, exactly 900 days and so on.

The term "about [...]", as used herein, encompasses the explicitly recited amount or number as well as deviations from the recited value of for example 15%, more preferably of 10%, and most preferably of 5% is encompassed by the term "about".
With regard to the number of days referred to herein, the skilled person is aware that such time spans are relative amounts of time that do not require a 100% accuracy as long as the time span approximately corresponds to the recited time span. Accordingly, the term "about" with regard to days refers for example to deviations of several hours and/or minutes. In other words, the time of treatment does not have to be exactly the recited amount of days but may differ by several minutes, such as for example 1 minute, 2 minutes, 3 minutes, 4 minutes, 5 minutes, 6 minutes, 7 minutes, 8 minutes, 9 minutes, 10 minutes etc.. Further, the time of treatment may differ by several hours from the recited amount of days, such as for example 1 hour, 2 hours, 3 hours, 4 hours, 5 hours, 6 hours, 7 hours, 8 hours, 9 hours, 10 hours or even 11 hours. As an example, the term "about 2 days" encompasses exactly two days, i.e. 48 hours, but also encompasses for example 41 hours, 42 hours, 43 hours, 44 hours, 45 hours, 46 hours, 47 hours, 49 hours, 50 hours, 51 hours, 52 hours, 53 hours, 54 hours or 55 hours. It will be appreciated that the deviation may also be of e.g. 1 hour and 10 minutes or any other combination of the above recited minutes and hours.

In another preferred embodiment of the method of the invention, the cell preparation is obtained from fetal or neonatal organs.

In another preferred embodiment of the method of the invention, the cell population is obtained from lung, liver or spleen.

In a further preferred embodiment of the method of the invention, the mammal is selected from the group consisting of rodents, humans, dogs, felids, pigs, ruminants or primates. Preferably, the mammals are animals routinely used in laboratory research, such as e.g. mice, rats, dogs and non-human primates.

Non-limiting examples of "rodents" are mice, rats, squirrels, chipmunks, gophers, porcupines, beavers, hamsters, gerbils, guinea pigs, degus, chinchillas, prairie dogs, and groundhogs. Preferably, the rodents are selected from the group consisting of mice and rats. More preferably, the mice are selected from wild-type mice of any background, such as e.g. BALB/c, C57BL/6, C57BL/10 or 129Sv mice, or the mice are selected from modified animal models such as e.g. TLR4-deficient C57BL/10ScN, TLR2 deficient C57BL/6, TLR9 deficient C57BL/6 or human TLR4-transgenic C57BL/10ScN mice, Syk deficient C57BL/6, TRIF deficient C57BL/6, Myd88 deficient C57BL/6, Unc93b1 deficient C57BL/6, CD14 deficient 129/sv, IL-1 Receptor deficient C57BL/6, IRF3 deficient C57BL/6 as well as IRF7 deficient C57BL/6.

Non-limiting examples of "dogs" include members of the subspecies *canis lupus familiaris* as well as wolves, foxes, jackals, and coyotes. Preferably, the dogs are from the subspecies *canis lupus familiaris* and in particular are selected from beagles or Dobermans.

Non-limiting examples of "felids" include members of the two subfamilies: the pantherinae, including lions, tigers, jaguars and leopards and the felinae, including cougars, cheetahs, servals, lynxes, caracals, ocelots and domestic cats.

Examples of "ruminants" include, without being limiting, cattle, goats, sheep, giraffes, bison, moose, elk, yaks, water buffalo, deer, camels, alpacas, llamas, antelope, pronghorn, and nilgai. Preferably, the ruminants are selected from the group consisting of cattle, goats and sheep.

The term "primates", as used herein, refers to all monkeys including for example cercopithecoid (old world monkeys) or platyrrhine (new world monkeys) as well as lemurs, tarsiers, apes and marmosets (Callithrix jacchus). Preferably, the primates are selected from the group consisting of marmosets as well as guenons, macaques, capuchins and squirrel monkeys.

It will be appreciated that the cells obtained from the tissue are to be plated at an appropriate starting density. For example, cells are seeded at a starting density of between at least 1 and 1.000.000.000 cells per ml of culture medium, such as e.g. between 100 and 500.000.000 cells per ml of culture medium, preferably between 1.000 and 100.000.000 cells per ml of culture medium cells per ml, such as e.g. between 10.000 and 10.000.000 cells per ml of culture medium. In a particularly preferred embodiment of the method of the present invention, the cells are plated at a starting density of between 100.000 (1×10⁵) and 1.000.000 cells per ml of culture medium. Preferably, the cell culture dish is not coated, such as e.g. with laminin. Moreover, the presence of feeder cells is not required, i.e. the cells of the present invention are preferably cultured in the absence of feeder cells.

In another preferred embodiment of the method of the invention, the cell culture comprises the steps (a) culturing the cell preparation for 4 to 10 days, preferably 5 to 9 days, more preferably 6 to 8 days and most preferably 7 days (b-i) diluting and transferring the non-adherent cells to (a) new cell culture vessel(s); (b-ii) optionally treating the adherent cells left in the original cell culture vessel with 1.5mM EDTA/PBS for approx. 10 minutes at 37°C and transferring the detached cells to (a) new cell culture vessel(s); (c) culturing the transferred cells for 4 to 10 days, preferably 5 to 9 days, more preferably 6 to 8 days and most preferably 7 days and (d) repeating steps (b-i), (b-ii) and (c) at least five times. The cell culture conditions and media are as defined herein above and GM-CSF is present in accordance with the method of the invention, more preferably GM-CSF is present throughout all cell culture steps recited in accordance with this preferred embodiment.

The dilution of the non-adherent cells in step (b-i) is preferably a 3- to 5-times dilution of the non-adherent cells, i.e. the amount (e.g. in ml) of the cells and medium obtained from the original cell culture vessel is determined and this amount is multiplied by e.g. 3 or 5 to determine the amount to be used in the diluted culture. For example, if 1ml of cells and medium is obtained from the original cell culture, then 2ml (for a 3-fold dilution) or 4ml (for a 5-fold dilution) of fresh medium is added to said 1ml for dilution.

The term "about" has been defined herein above and applies *mutatis mutandis* also to this embodiment.

The term "at least five times" in step (d) refers to exactly five times as well as more than five times, such as e.g. six times, seven times, eight times, nine times, ten times, eleven times, twelve times, 13 times, 14 times, 15 times, 16 times, 17 times, 18 times, 19 times, 20 time or more. However, after repeating steps (b-i), (b-ii) and (c) at least five times, the cells are self-renewing, non-transformed macrophages that do not require the above described culture steps (b-i), (b-ii) and (c) any longer and, consequently, may be cultured using any suitable cell culture known in the art or described herein.

In accordance with this embodiment, the cells are cultured under the conditions and in the medium as defined herein above. Non-adherent and only loosely adherent cells are repeatedly transferred to new culture vessels, thus removing contaminating cells such as e.g. fibroblasts which adhere more quickly to the cell culture vessel than the developing self-renewing, non-transformed macrophages. This preferred embodiment is of particular relevance when the cells are obtained from lung.

In accordance with the method of the invention, the self-renewing, non-transformed macrophages are characterised by the expression of at least four markers selected from the group consisting of GM-CSF receptor, M-CSF receptor, CD11c, TLR2, TLR4, TLR3, F4/80, CD16, CD11b, CD14 and Marco.

All of the marker proteins referred to herein are defined in accordance with the pertinent prior art.

The term "GM-CSF receptor", as used herein, refers to the complex of proteins mediating the binding of GM-CSF to the GM-CSF receptor and the elicitation of signal transduction. Engagement of the GM-CSF receptor leads to the activation of various kinases leading to the induction of the c-myc, pim-1, PU.1 and STAT5 transcription factors (Hercus et al., 2009; Shibata et al., 2001). The human α-chain of the GM-CSF receptor is represented by the GenBank accession number AAA35909 (publication date June 8, 1993), the murine α-chain of the GM-CSF receptor is represented by the NCBI Reference Sequence: NP_034100.2 (publication date October 22, 2011) and the rat α-chain of the GM-CSF receptor is represented by NCBI Reference Sequence: NP_001032749.1 (publication date November 11, 2011). The human β-chain of the GM-CSF receptor is represented by the GenBank accession number AAA18171.1 (publication date May 16, 1994), the murine β-chain of the GM-CSF receptor is represented by the NCBI Reference Sequence: NP_031806.3 (publication date April 11, 2011) and the rat human β-chain of the GM-CSF receptor is represented by NCBI Reference Sequence: NP_598239.1 (publication date November 12, 2011). The GM-CSF receptor is well known in the art and has been described for example in Rasko and Gough 1994.

"M-CSF receptor", as used throughout the present invention, refers to the complex of proteins mediating the binding of M-CSF (CSF-1) to the M-CSF receptor and the elicitation of signal transduction. M-CSF triggers signalling through receptor phosphorylation and the binding of the Grb2 SH2 domain, the SH3/SH2 adapter protein termed Mona, the PI-3 kinase (*PI3*-*K*) and the N-SH2 domain of phospholipase Cy2 (*PLCy2*) proteins and stimulates the proliferation and maturation of macrophages. The human M-CSF receptor is represented by the GenBank accession number AAB51696.1 (publication date October 12, 2005), murine M-CSF receptor by the GenBank accession number AAH43054.1 (publication date July 15, 2006) and rat M-CSF receptor by NCBI Reference Sequence: NP_001025072.1 (publication date March 26, 2012). The M-CSF receptor is well known in the art and has been described for example in Chitu V and Stanley ER; Colony-stimulating factor-1 in immunity and inflammation; Curr Opin Immunol. 2006 Feb;18(1):39-48 and Barreda DR, Hanington PC, Belosevic M; Regulation of myeloid development and function by colony stimulating factors; Dev Comp Immunol. 2004 May 3;28(5):509-54.
"CD11c", as used throughout the present invention, refers to the integrin alpha-X protein, an adhesion molecule mediating cell to cell binding and immune cell recruitment. Human CD11c is represented by the NCBI Reference Sequence: NP_000878.2 (publication date March 24, 2012; this is the precursor, the mature protein consists of amino acids 20 to 1163 of this sequence), murine CD11c by the GenBank accession number AAH57200.1 (publication date March 30, 2005) and rat CD11c by NCBI Reference Sequence: XP_574569.3 (publication date April 2, 2010). CD11c is well known in the art and has been described for example in Barthel SR, Johansson MW, McNamee DM, Mosher DF; Roles of integrin activation in eosinophil function and the eosinophilic inflammation of asthma; J Leukoc Biol. 2008 Jan;83(1):1-12.

The term "TLR", as used herein, refers to the Toll Like Receptors, in particular TLR2, TLR4 and TLR3. Toll Like Receptors are involved in innate activation and the production of inflammatory mediators as reviewed e.g. in Kawai and Akira 2011.
Human TLR4 is represented by the GenBank accession number CAH72619.1 (publication date January 13, 2009), murine TLR4 by the GenBank accession number EDL31078.1 (publication date June 7, 2007) and rat TLR4 by NCBI Reference Sequence: NP_062051.1 (publication date June 3, 2012).
Human TLR2 is represented by the GenBank accession number AAH33756.1 (publication date October 7, 2003), murine TLR2 by the GenBank accession number AAF04277.1 (publication date October 27, 1999) and rat TLR2 by NCBI Reference Sequence: NP_942064.1 (publication date May 13, 2012).
Human TLR3 is represented by the GenBank accession number ABC86910.1 (publication date January 3, 2011), murine TLR3 by the GenBank accession number AAH99937.1 (publication date July 15, 2006) and rat TLR3 by GenBank accession number BAC81504.1 (publication date August 5, 2003).

The term "F4/80", as used herein, refers to F4/80 protein in mice, which corresponds to the Emr1 protein in humans, which activates a G-protein coupled receptor signaling pathway and is involved in the generation of antigen-specific efferent regulatory T (T reg) cells that suppress antigen-specific immunity, as described e.g. in Lin *et al*. 2005. In mice, F4/80 is represented by the GenBank accession number AAH75688.1 (publication date July 28, 2005) and in rats F4/80 is represented by GenBank accession number AAU95564.1 (publication date December 3, 2004).

"CD16", as used herein, refers to the low affinity IIIa receptor, which is a receptor for the Fc portion of immunoglobulin G, and is involved in the removal of antigen-antibody complexes and in other antibody-dependent responses. Aberrant expression of FcyRIIIA (CD16) contributes to the development of atherosclerosis, see e.g. Huang Y, *et al*. 2012. Human CD16 is represented by the GenBank accession number AAH36723.1 (publication date July 15, 2006), murine CD16 by the NCBI Reference Sequence: NP_653142.2 (publication date March 8, 2012) and rat CD16 by NCBI Reference Sequence: NP_653142.2 (publication date March 8, 2012).

"CD11b", as used throughout the present invention, refers to the integrin beta 2 alpha subunit (itgam) protein and is an adhesion molecule mediating cell to cell binding and immune cell recruitment. CD11b is well known in the art, see e.g. Barthel *et al*. 2008 and Janeway *et al.* 2001. Human CD11b is represented by the NCBI Reference Sequence: NP_000623.2 (publication date May 6, 2012), murine CD11b by the NCBI Reference Sequence: NP_001076429.1 (publication date June 2, 2012) and rat CD11b by NCBI Reference Sequence: NP_036843.1 (publication date November 11, 2011).

As used herein, the term "CD14" refers to monocyte differentiation antigen CD14. It is the co-receptor for LPS, transfers LPS to TLR4 and contributes to cytokine production in response to endotoxin; see e.g. Freudenberg *et al*. 2008. Human CD14 is represented by the NCBI Reference Sequence: NP_001167576.1 (publication date May 26, 2012), murine CD14 by the NCBI Reference Sequence: NP_033971.1 (publication date June 3, 2012) and rat CD14 by NCBI Reference Sequence: NP_068512.1 (publication date April 21, 2012).

"MARCO", as used throughout the present invention, refers to macrophage receptor with collagenous structure (MARCO), which is a scavenger receptor important to phagocytose microbes and other environmental particles; see e.g. Mukhopadhyay 2011. Human MARCO is represented by the NCBI Reference Sequence: NP_006761.1 (publication date April 22, 2012), murine MARCO by the NCBI Reference Sequence: NP_034896.1 (publication date May 12, 2012) and rat MARCO by NCBI Reference Sequence: NP_001102481.1 (publication date June 15, 2012).

In accordance with the invention, encompassed are also at least five, at least six, at least seven, such as at least eight or all nine of the recited markers as well as exactly four, exactly five, exactly six, exactly seven, exactly eight or exactly nine markers from the recited list of markers. Preferably, one of said at least four markers is MARCO. For example, the at least four markers may be a combination of MARCO, F4/80, TLR4 and TLR2. Preferably, the at least four markers are marker proteins of either humans, mice or rats and more preferably the markers proteins are proteins of the same animal species as the cel.

Preferably, the self-renewing, non-transformed macrophages are a population of cells comprising at least 70% of cells expressing four or more of the above defined markers, more preferably at least 80%, such as at least 85%, such as at least 90%, such as at least 95%, more preferably at least 98%, even more preferably at least 99% and most preferably 100%. It is also particularly preferred that at least 98%, preferably 100% of the cells express all of the mentioned markers.

Suitable methods of determining whether four or more of the above recited markers are expressed by the cells are known in the art. Such methods include, without being limiting, determining the expression of a marker on the amino acid level as well as on the nucleic acid level as well as by assessing the functional response of the cell to certain stimuli, such as for example the cytokine response to LPS.
Methods for the determination of expression levels of a marker on the amino acid level include but are not limited to immunohistochemical methods but also e.g. FACS staining, western blotting or polyacrylamide gel electrophoresis in conjunction with protein staining techniques such as Coomassie Brilliant blue or silver-staining or identification of protein (for example after SDS-PAGE separation) by sequencing. Also of use in protein quantification is the Agilent Bioanalyzer technique. Further methods of determination include cell sorting approaches such as described herein above. Methods for determining the expression of a marker on the nucleic acid level include northern blotting, PCR, RT-PCR or real time PCR as well as techniques employing microarrays. All these methods are well known in the art and have been described in part in the appended examples.

In accordance with the method of the invention, the self-renewing, non-transformed macrophages are characterised by a lack of expression of at least three markers selected from the group consisting of B220, IgE receptor, T-cell surface glycoprotein CD3 epsilon chain, and B-cell receptor. It is also described herein thatwhere the cells are obtained from mice, the self-renewing, non-transformed macrophages are characterised by a lack of expression of at least three markers selected from the group consisting of MHC-II, B220, Gr1, IgE receptor, T-cell receptor and B-cell receptor.

As used herein, the term "MHC-II" refers to the major histocompatibility complex Class II molecules which present peptides derived from extracellular proteins to lymphocytes. MHC-II are important for the presentation of antigens in dendritic cells, B lymphocytes and macrophages; see e.g. Janeway *et al*. 2001. Human MHC-II is represented by the NCBI Reference Sequence: NP_001230891.1 (publication date June 2, 2012), murine MHC-II by the GenBank accession number AAF21604.3 (publication date September 20, 2006) and rat MHC-II by GenBank accession number AAV40612.1 (publication date November 12, 2004).

The term "B220", as used herein, refers to protein tyrosine phosphatase, receptor type, C protein, which is a marker for B-cells mediating B-cell signalling; see e.g. Welinder *et al*. 2011. Human B220 is represented by the GenBank accession number AAI48258.1 (publication date August 21, 2007), murine B220 by the NCBI Reference Sequence: NP_035340.2 (publication date June 9, 2012) and rat B220 by NCBI Reference Sequence: NP_001103357.1 (publication date April 22, 2012).

"IgE receptor", as used throughout the present invention, refers to the high affinity immunoglobulin epsilon receptor subunit alpha, which mediates the binding of IgE immunoglobulins to mastocytes; see e.g. Janeway *et al*. 2001. The human IgE receptor is represented by the NCBI Reference Sequence: NP_001992.1 (publication date June 2, 2012), murine IgE receptor by the NCBI Reference Sequence: NP_034314.2 (publication date May 26, 2012) and rat IgE receptor by NCBI Reference Sequence: NP_036856.1 (publication date April 21, 2012).

As used herein, the term "T-cell surface glycoprotein CD3 epsilon chain," refers to the T-cell surface glycoprotein CD3 epsilon chain, which is the main antigen binding protein on the surface of T lymphocytes and elicits T cell activation and which together with CD3-gamma, - delta and -zeta, and the T-cell receptor alpha/beta and gamma/delta heterodimers, forms the T cell receptor-CD3 complex; see e.g. Janeway *et al.* 2001. The human T-cell receptor is represented by the NCBI Reference Sequence: NP_000724.1 (publication date April 22, 2012), murine T-cell receptor by the NCBI Reference Sequence: NP_031674.1 (publication date June 9, 2012) and rat T-cell receptor by NCBI Reference Sequence: NP_001101610.1 (publication date November 12, 2011).

"B-cell receptor", as used throughout the present invention, refers to the B-cell antigen receptor complex-associated protein alpha chain, which is the main antigen binding protein on the surface of B lymphocytes and elicits B cell activation; see e.g. Janeway *et al*. 2001. The human B-cell receptor is represented by the NCBI Reference Sequence: NP_001774.1 (publication date May 5, 2012), murine B-cell receptor by the NCBI Reference Sequence: NP_031681.2 (publication date June 2, 2012) and rat B-cell receptor by NCBI Reference Sequence: XP_001060872.2 (publication date April 2, 2010).

The term "Gr1", as used herein, refers to the myeloid differentiation antigen Gr-1, a marker which is only present in mice. Gr1 is a marker of a subset of mouse leukocytes, as described e.g. in Janeway *et al*. 2001. Gr1 is represented by the NCBI Reference Sequence: XP_001475803.2 (publication date February 23, 2012).

All of the definitions provided herein above for markers expressed by the macrophages in accordance with the invention, in particular the methods referred for determining the presence or absence of marker expression, apply *mutatis mutandis* to these markers that are lacking expression in the macrophages in accordance with the invention. Moreover, it is preferred that in the macrophage cell population of the present invention less than 30% of the cells express three or more of the markers defined in this embodiment, more preferably less than 20%, such as less than 15%, such as less than 10%, such as less than 5%, more preferably less than 3%, even more preferably less than 1% and most preferably 0%.

In accordance with this embodiment, encompassed are also at least four, at least five or all six of the recited markers as well as exactly three, exactly four, exactly five or exactly six markers from the recited list of markers. Preferably, one of said at least three markers is a T or B cell receptor. For example, the at least three markers may be a combination of T cell receptor, B cell receptor and IgE receptor. Preferably, the at least three markers are marker proteins of either humans, mice or rats and more preferably the markers proteins are proteins of the same animal species as the cells.

The macrophages obtained by the method of the present invention and expressing the above recited markers or marker combinations most closely resemble lung alveolar and spleen marginal zone macrophages. Thus, the macrophages of the present invention are alveolar macrophage-like cells or spleen marginal zone macrophage-like cells. The terms "alveolar macrophage-like cells" and "spleen marginal zone macrophages" refers to cells characterised by the expression of at least four markers selected from the group consisting of GM-CSF receptor, M-CSF receptor, CD11c, TLRs, F4/80, CD16, CD11b, CD14 and MARCO and/or a lack of expression of at least three markers selected from the group consisting of B220, Gr1, IgE receptor, T-cell receptor and B-cell receptor, as defined herein above.
The method of the invention may further comprise introducing an immortalisation gene into the cells obtained by the method of the invention. Suitable genes for the immortalisation of macrophages include for example STAT5 or the SV40 large T antigen as has been described in the art, e.g. in Mbawuike IN, Herscowitz HB, MH-S, a murine alveolar macrophage cell line: morphological, cytochemical, and functional characteristics., J Leukoc Biol. 1989 Aug;46(2):119-27. In another preferred embodiment of the method of the invention, the method of the present invention further comprises introducing a constitutively active form of STAT5 into the cells obtained by the method of the invention.

The term "introducing" as used in accordance with the present invention relates to the process of bringing the recited nucleic acid sequence into the target cell. Methods for introducing nucleic acid sequences are well known to the skilled person and include, without being limiting, transfections based on lipofection, electroporation, nucleofection, magnetofection or viral vector infection. Preferably, the method should be a method achieving high transfection efficiency. For example, transfection efficiencies of at least 30 %, such as at least 50 %, or at least 80 % are preferred. Preferably, retroviral vectors are used to achieve transfection as said vectors not only mediate efficient entry of the nucleic acid sequences into the cells but also their integration into the genomic DNA of the target cell. Retroviral vectors have been shown to be suitable for the transfection of a wide range of cell types from different animal species, to integrate genetic material carried by the vector into the respective cells, to express the transfected coding sequences at high levels, and, advantageously, retroviral vectors do not spread or produce viral proteins after their use in such transfection methods. Suitable retroviral vector systems are well-known to the person skilled in the art such as, e.g., retroviral vectors with the MoMuLV LTR, the MESV LTR, lentiviral vectors with various internal promoters like the CMV promoter. Episomal vector systems like adenovirus vectors, other non-integrating vectors as well as episomally replicating plasmids can also be used. Preferably, the retroviral pMYs vector system is used in the method of the invention (see e.g. Sekine R, Kitamura T, Tsuji T, Tojo A., Efficient retroviral transduction of human B-lymphoid and myeloid progenitors: marked inhibition of their growth by the Pax5 transgene. Int J Hematol. 2008 May;87(4):351-62).
"STAT5" is defined in accordance with the pertinent art and relates to the signal transducer and activator of transcription 5 A and 5B and has been described in the art, e.g. in Pullen et al., Novel mechanism for Fc{epsilon}RI-mediated signal transducer and activator of transcription 5 (STAT5) tyrosine phosphorylation and the selective influence of STAT5B over mast cell cytokine production., J Biol Chem. 2012 Jan 13;287(3):2045-54.
Mouse STAT5 is represented by the NCBI Reference Sequences: NP_035619.3 (publication date June 2, 2012) and its mRNA is represented by NCBI Reference Sequence: NM_011488.3 (publication date June 2, 2012). Human STAT5 is represented by the NCBI Reference Sequences: NP_036580.2 (publication date May 6, 2012) and its mRNA is represented by NCBI Reference Sequence: NM_003152.3 (publication date May 6, 2012).

The term "constitutively active", as used herein, is defined in accordance with the pertinent art and relates to the active form of the transcription factor protein that translocates to the nucleus and activates gene expression without the need for any stimulatory signal. The constitutively active STAT5 transcription factor is well known in the art and has been described e.g. in Heltemes-Harris *et al*. 2001.

Due to the introduction of a gene encoding a constitutively active form of STAT5 into the cells of the present invention, these cells can be rendered independent from the presence of GM-CSF in the culture medium. Apart from the omission of GM-CSF from the culture medium, all of the above described cell culture conditions apply *mutatis mutandis* also to the cell culture of these modified cells. It will be appreciated that after introduction of the constitutively active STAT5, these cells are no longer "non-transformed" cells. Accordingly, the macrophages of the present invention which have been further modified by the introduction of the constitutively active STAT5 are referred to herein as the "transformed macrophages of the invention" or the "modified macrophages of the invention".

In another preferred embodiment of the method of the invention, the method further comprises isolating a single cell obtained by the methods of the invention and clonally expanding said cell.

The term "isolating" refers to the separation of a single cell from the initial culture dish and its transfer to a new culture vessel, such as for example a different cell culture dish or flask. Methods of isolation of cells are well known in the art and include, without being limiting, mechanical isolation, limited dilution as well as cell sorting approaches. Mechanical isolation relates to the selection and isolation of a cell by e.g. manual picking of the cell, where necessary under a microscope, automated picking by use of a robot or laser-capture microdissection. Manual picking of a cell may be performed by methods known in the art, such as for example aspiration of the cell into the tip of a pipette. Cell sorting approaches have been described herein above and include, for example, magnetic activated cell sorting (MACS), flow cytometry activated cell sorting (FACS) or panning approaches. Such methods for cell isolation are well known in the art and have been described, e.g. in Dainiak et al., ((2007) Adv Biochem Eng Biotechnol. 2007;106:1-18), Murray ((2007) Acta Histochemica 109:171-176) or Tung et al. ((2007) Clin Lab Med. 27:453-468).

The term "expanding", in accordance with the present invention, refers to a multiplication of cells, thus resulting in an increase in the total number of cells. For example, cells can be expanded to at least 100 times their original number, such as at least 1.000 times their original number, preferably at least 10.000 times their original number, such as at least 100.000 times their original number etc.. Expansion of cells may be achieved by any known method, e.g. by culturing the cells under appropriate conditions to high density or confluence and subsequently splitting (or passaging) of the cells, as described herein above.

The term "clonally expanding" refers to the fact that one single cell is expanded to a plurality of cells, thus resulting in a cell culture of clones of the initial cell.

In a further preferred embodiment of the method of the invention, the self-renewing, non-transformed macrophages obtained are free or substantially free of pathogens.

Pathogens to be avoided are well known to the skilled person and include, without being limiting, viruses such as for example Hepatitis virus A, B, C, Epstein-Barr-Virus or HIV-Virus and bacteria such as for example mycoplasm or chlamydia. The cells are considered to be essentially free of pathogens if no more than 1 in 10⁴ cells comprises pathogens, such as e.g. no more than 1 in 10⁶ cells, preferably no more than 1 in 10¹⁰ cells, such as e.g. no more than 1 in 10¹⁵ cells, such as no more than 1 in 10²⁰ cells, more preferably no more than 1 in 10³⁰ and most preferably no more than 1 in 10⁵⁰ cells.

The present invention further relates to macrophages obtainable by the method of the invention, wherein the macrophages are self-renewing, non-transformed macrophages being characterised by the expression of at least four markers selected from the group consisting of GM-CSF receptor, M-CSF receptor, CD11c, TLR2, TLR4, TLR3, F4/80, CD16, CD11b, CD14 and Marco, and being characterised by a lack of expression of at least 3 markers selected from the group consisting of B220, Gr1, IgE receptor, T-cell surface glycoprotein CD3 epsilon chain and B-cell receptor.

To the inventors' best knowledge, such cells do not occur naturally. Although primary alveolar macrophages are similar to cells obtained in accordance with the present invention in terms of certain surface markers and similar biological response, they differ in the expression of other markers, most prominently the lack of MHC II in the cells obtained by the method of the present invention. In addition, alveolar macrophages are not capable of replicating indefinitely in culture.

The present invention further relates to the macrophages of the invention for use in medicine or medical/pharmaceutical research.

The macrophages of the invention as well as a composition comprising the macrophages of the invention can be used in a variety of experimental as well as therapeutic scenarios, such as e.g. gene therapy, cell therapy or drug screening.

Gene therapy, which is based on introducing therapeutic DNA constructs for correcting a genetic defect into cells by *ex vivo* or *in vivo* techniques, is one of the most important applications of gene transfer. Suitable vectors and methods for *in vitro* or *in vivo* gene therapy are described in the literature and are known to the person skilled in the art (Davis PB, Cooper MJ., AAPS J. (2007), 19;9(1):E11-7; Li S, Ma Z., Curr Gene Ther. (2001),1(2):201-26). Accordingly, macrophages obtained from a sample from a patient could, for example, be genetically corrected by methods known in the art and subsequently be administered to the patient. This evidences the applicability of the macrophages of the invention in gene therapy and/or cell therapy.
The macrophages of the invention can also be used to identify drug targets and test potential therapeutics, in particular for the treatment of respiratory diseases, hence reducing the need for primary cell cultures and *in vivo* studies. Non-limiting examples of respiratory diseases include asthma, asbestosis, silicosis, COPD and pneumonia. Experimental setups and methods to identify and/or assess effects of a potential drug including, for example, target-site and -specificity, toxicity or bioavailability are well-known to the person skilled in the art. Further, macrophages of the invention may also be useful in experimental settings - besides therapeutic applications - to study a variety of aspects related to macrophage activity. The macrophages can further be subject to studies relating to, e.g. gene targeting, differentiation studies, tests for safety and efficacy of drugs, transplantation of autologous or allogeneic regenerated macrophages, apoptosis or diseases like, e.g., inflammatory disease, bacterial , viral , fungal, protozoal infections (tuberculosis, listeriosis, salmonellosis, staphylococcus, streptococcus, helicobacter, adenovirus, poxvirus, morbilli virus, hepatitis A, B, C, E virus, herpes infections, Aspergillus, Candida, Pneumocystis, Leshmania, Trypanosoma, Toxoplasma infections), helminthiasis, asthma, eczema, contact allergies and contact dermatitis as well as the side effects associated with virus vector gene-therapy.

Accordingly, the present invention further relates to a method for identifying a compound having a pharmacological, differentiating, proliferative or anti-proliferative, cytotoxic or transforming effect on the macrophages obtained or obtainable by the method of the invention, or cells derived therefrom, comprising: (a) contacting the macrophages obtained or obtainable by the method of the invention, or cells derived therefrom, with a test compound; and (b) determining whether the test compound has one or more of said effects on said macrophages or cells derived therefrom.

In accordance with the present invention, the test compound may be selected from the group consisting of nucleic acids, such as DNA, cDNA, RNA, dsRNA, siRNA, shRNA, miRNA, proteins, peptides, sugars, lipids, small molecules (organic or inorganic), or any combination thereof.

Depending on the nature of the test compound, different step of contacting the cells may need to be adjusted. For example, if expressible proteins or peptides are to be evaluated, the corresponding coding sequences may be introduced as described herein above. In contrast, small molecules may be introduced by exogenously adding the respective compound to the cell medium and taking advantage of passive or active cellular uptake mechanisms. The skilled person is well-aware of methods of contacting cells with a test compound.

The function of test compound may be identified and/or verified by using high throughput screening assays (HTS). High-throughput assays, independently of being biochemical, cellular or other assays, generally may be performed in wells of microtiter plates, wherein each plate may contain, for example 96, 384 or 1536 wells. Handling of the plates, including incubation at temperatures other than ambient temperature, and bringing into contact of test compounds with the cell culture is preferably effected by one or more computer-controlled robotic systems including pipetting devices. Where large libraries of test compounds are to be screened and/or screening is to be effected within short time, mixtures of, for example 10, 20, 30, 40, 50 or 100 test compounds may be added to each well. In case a well exhibits an activity on the cells, said mixture of test compounds may be de-convoluted to identify the one or more test compounds in said mixture giving rise to the observed activity.

The term "cells derived therefrom", as used herein, encompasses cells that were generated from the macrophages of the invention by genetical engineering, including the above defined "modified macrophages of the invention".

The term "genetical engineering", as used herein, refers to the process of modifying the genetic information of the cell, for example by bringing into a cell nucleic acid sequences that are not present in said cell prior to the step of genetic engineering (i.e. exogenous nucleic acid molecules) or by bringing into a cell nucleic acid sequences that are present in said cell prior to the step of genetic engineering (i.e. endogenous nucleic acid molecules), thereby, for example, increasing the expression of the expression product encoded by said nucleic acid molecule. The cells prior to genetic engineering are also referred to herein as "parental cells". Non-limiting examples of exogenous and/or endogenous nucleic acid molecules include those encoding receptors or downstream signalling molecules involved in macrophage activity that are either not present or are not expressed in the parental cell line. For example, the macrophages of the present invention can be obtained from e.g. mice deficient for one or more TLRs, such as e.g. knock-out mice and can be genetically engineered to express one or more human TLRs. Such genetically engineered cells enable the study of human signal transduction pathways without the need to obtain macrophages from humans.
Additional examples of exogenous nucleic acid molecules include, without being limiting, molecules that inhibit the expression of a particular expression product, such as receptors or signalling molecules. Such inhibitory molecules include for example antisense nucleic acid molecules, small interfering RNAs (siRNAs), short hairpin RNAs (shRNAs) and microRNAs (miRNAs). The terms "antisense nucleic acid molecule", "small interfering RNAs (siRNAs)", "short hairpin RNAs (shRNAs)" and "microRNAs (miRNAs)", as used herein, are known in the art and are defined in accordance with the pertinent art.

Means and methods for introducing a nucleic acid molecule into a cell have been described herein above with regard to the introduction of a constitutively active form of STAT5. These definitions and preferred embodiments apply *mutatis mutandis* also to the genetical engineering in accordance with the present invention.

As described herein above, the macrophages obtained by the method of the invention are suitable for pharmacological or scientific research and may replace testing of macrophages directly obtained from a subject. In this regard, compounds may be tested with regard to their effect on macrophages that have been obtained by the method of the invention. Such effects comprise pharmacological, cytotoxic, proliferative or anti-proliferative, transforming or differentiating effect. The skilled person is well aware of methods to assess whether a test compound has an effect on the macrophages of the invention, for example by comparing macrophages cultured in the presence and absence of the test compound. Depending on the effect to be assessed the methods vary and may include, e.g., visual control by microscopy, measuring cell proliferation, apoptosis, cell morphology, cell migration, gene expression, cellular or secreted protein levels, the presence of lipid mediators (for example prostaglandins or leukotriens) or metabolic processes. The macrophages for use in this method are the self-renewing, non-transformed macrophages obtainable in accordance with the method of the invention or cells derived from these self-renewing, non-transformed macrophages of the invention. Such cells include for example the cells described herein above that are transfected with a constitutively active form of STAT5.

For example, the cells of the present invention may be employed as biosensors to detect unwanted impurities in a composition for administration. For example, impurities that are toxic for humans, such as bacterial endotoxin (LPS), may thus be identified. Furthermore, the cells of the present invention may be employed to screen potential new inhibitors of signalling pathways triggered by pathogens, allergens or toxins. As another example, the cells of the present invention may also be employed to produce glycosylated recombinant proteins, such as for example cytokines.

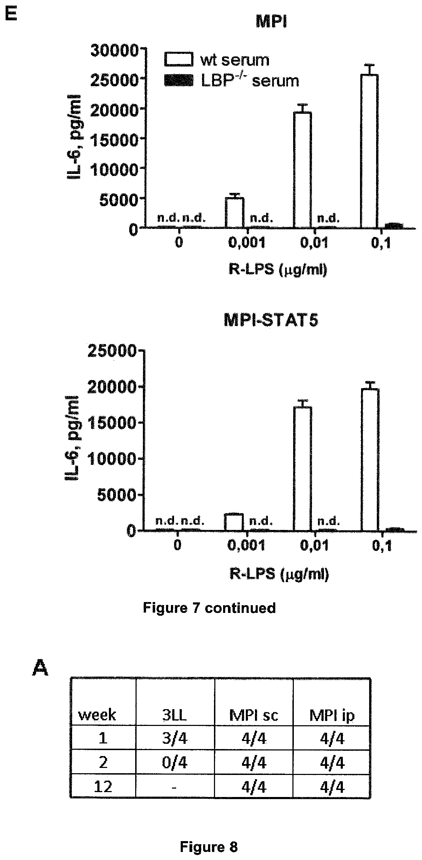

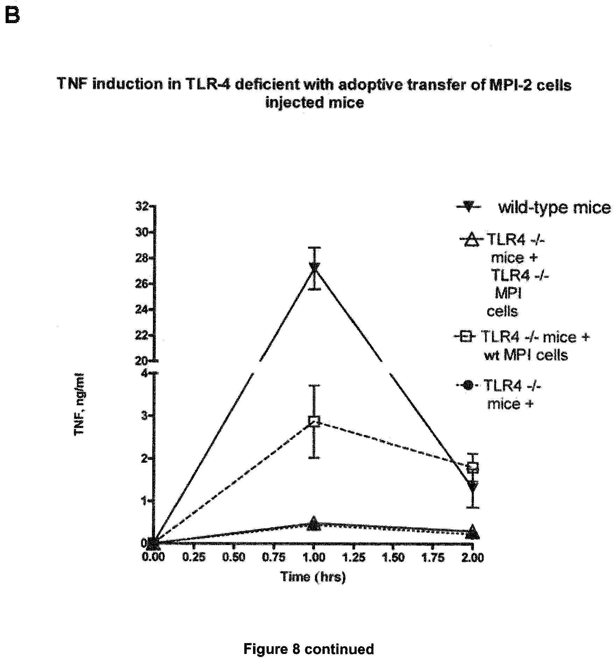

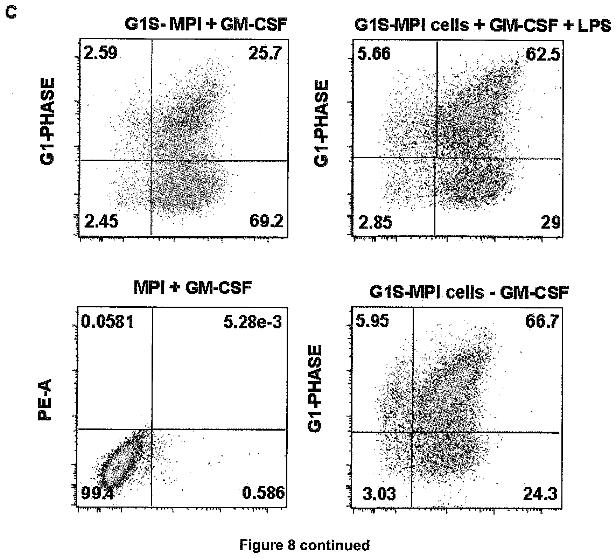

As another example, the cells of the present invention can be employed to study the role of macrophages *in vivo*. Cells obtained in accordance with the present invention from wild type or gene deficient mice can for example be injected into an animal deficient for a certain function and the responses mediated by the cells of the present invention cells can be studied. For example, mice deficient for TLR4 do not normally respond to LPS; however, TLR4 deficient mice injected with wt MPI cell do, as shown in Example 12 and Figure 8.

Unless otherwise defined, all technical and scientific terms used herein have the same meaning as commonly understood by one of ordinary skill in the art to which this invention belongs. In case of conflict, the patent specification, including definitions, will prevail.

The figures show:

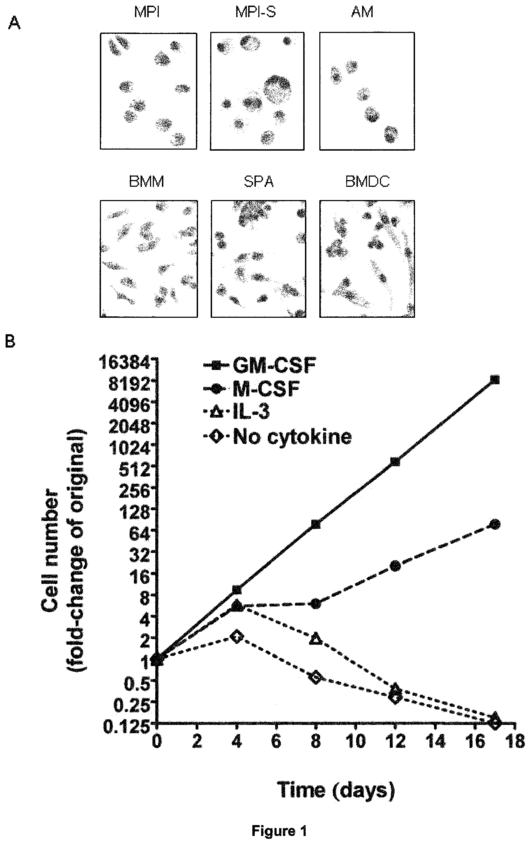

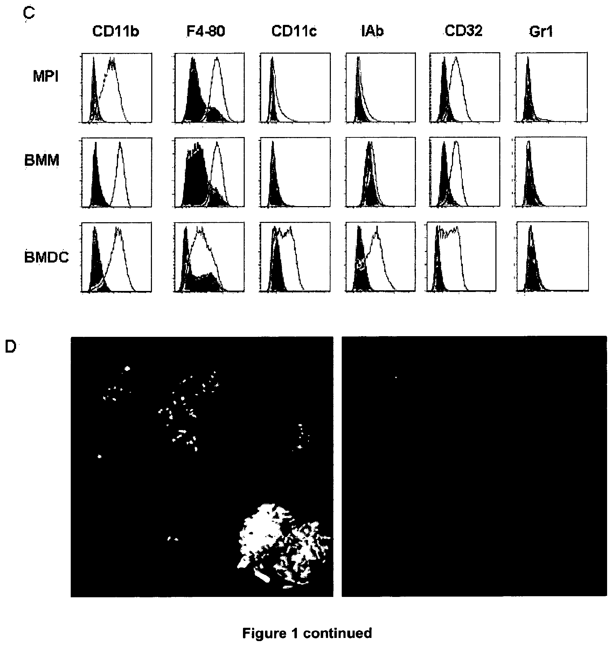

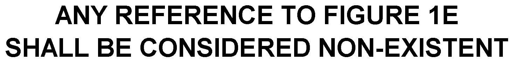

**Figure 1****:** MPI cells are factor dependent, self-renewing non transformed phagocytes. Giemsa staining of MPI cells from fetal liver (MPI) and spleen (MPI-S), alveolar macrophages (AM), bone marrow derived macrophages (BMM), SP37A3 cells (SPA) and bone marrow derived dendritic cells (BMDC) (A). Growth curve of MPI cells in the presence of GM-CSF, MCSF, IL-3 or without any growth factor (B). FACS staining of MPI cells, BMMs and BMDCs with control isotype antibody or myeloid surface marker specific antibodies (C). Visualisation of stained *P*. *acnes* bacteria white in DAPI stained MPI cells grey using confocal microscopy (D). MPI cells incubated with bacteria (left panel) or mock treated (right panel). A single P. acnes incubated cell is shown at high power as an insert.

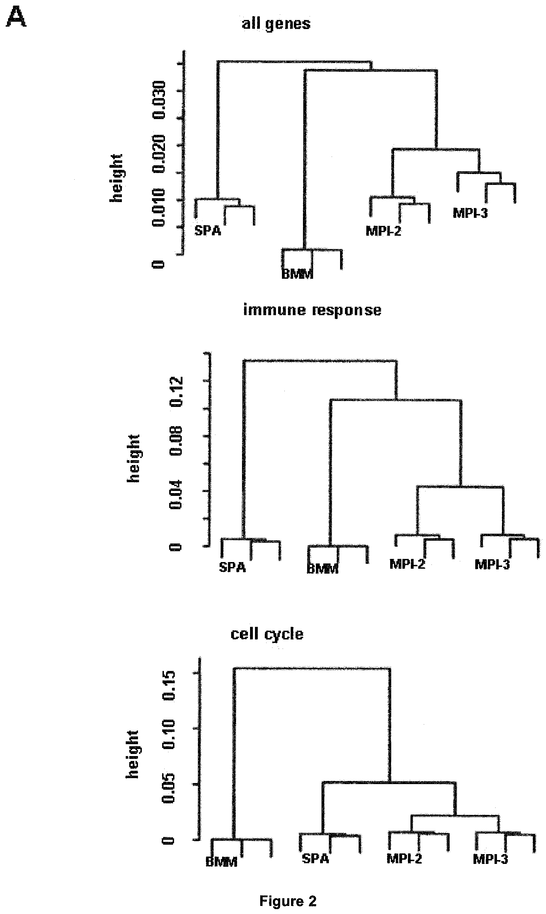

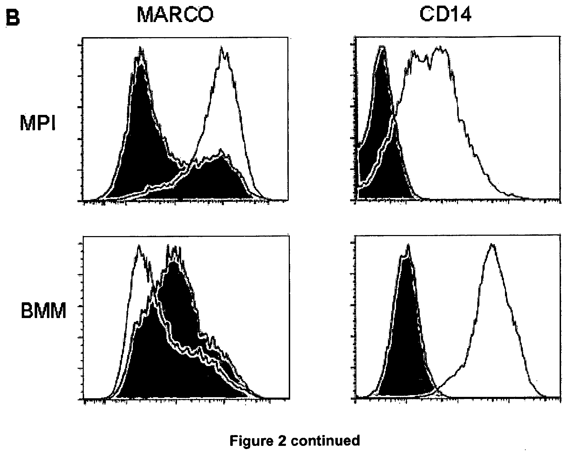

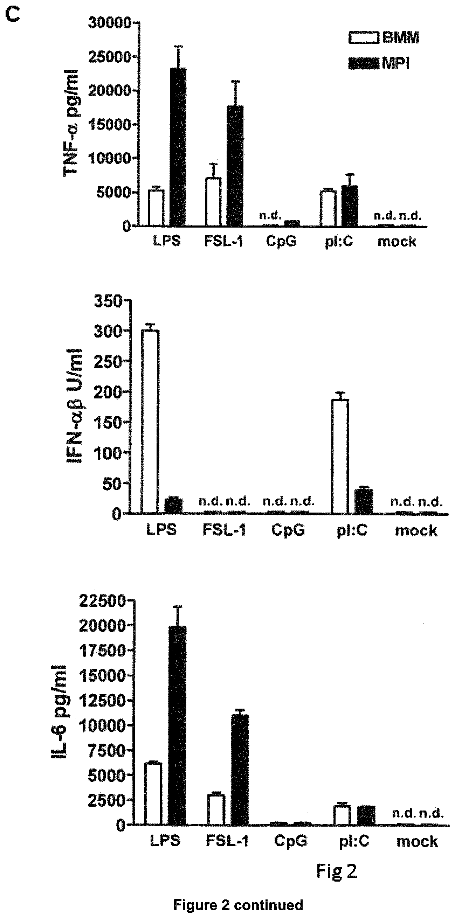

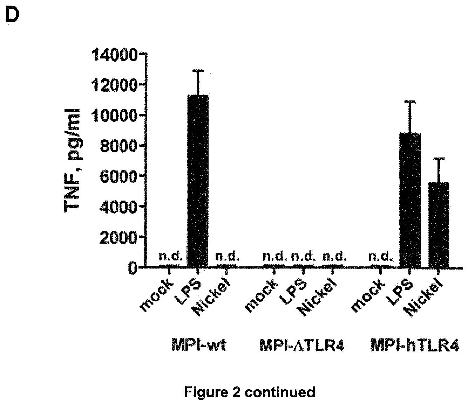

**Figure 2****:** Comparison of MPI cells and BMMs. Analysis of global gene expression in SP37A3 cells (SPA), BMMs and two independently made MPI cell lines (MPI-2 and MPI-3) with microarrays. Cluster analysis for all genes, immune response genes and cell cycle genes (A). Surface expression of MPI cells and BMMs using MARCO and CD14 specific (blue) or isotype control antibodies (red) (B). TNF-α, IL-6 and IFN-αβ levels of cell free supernatants of MPI cells and BMMs stimulated with 0.1 µg/ml smooth form LPS, 0.1 µg/ml lipopeptide FSL, CpG ODN 1668 and 25 µg/ml poly I:C (C). TNF-α levels of wt (MPI-wt), TLR4 deficient (MPI-ΔTLR4) and mouse TLR4 deficient, human TLR4 expressing (MPI-hTLR4) MPI cells in response to R-LPS (0.1 µg/ml) and nickel chloride (mM) (D).

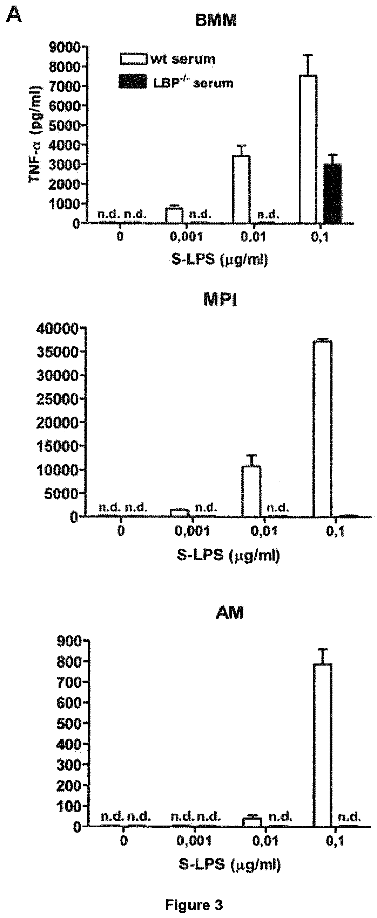

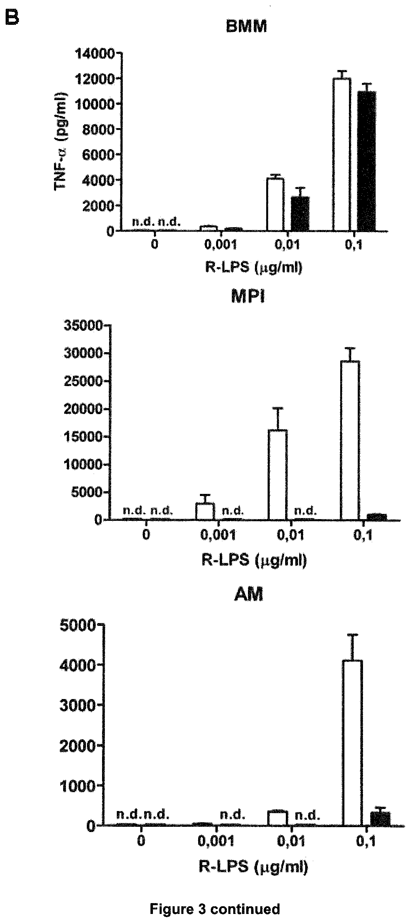

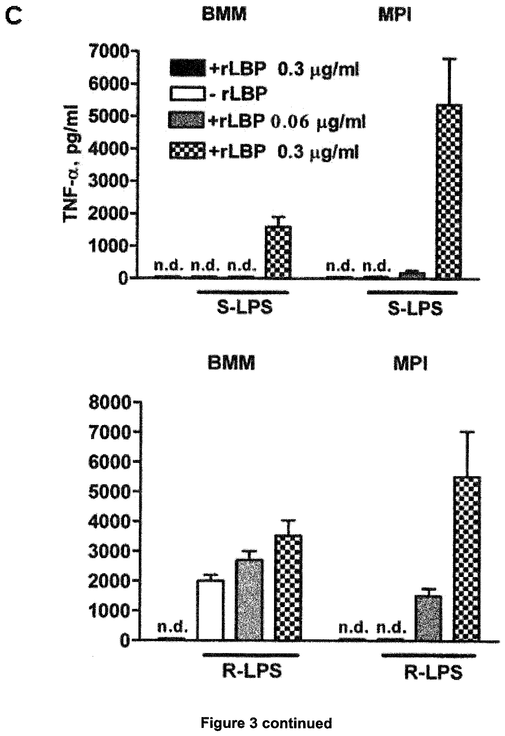

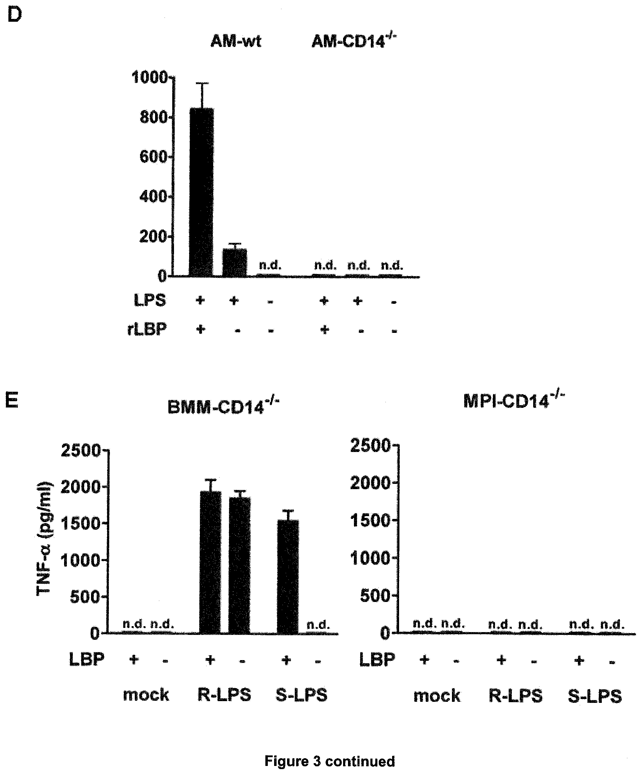

**Figure 3****:** MPI cells and alveolar macrophages require LBP and CD14 to sense rough form LPS. TNF-α levels of BMMs, MPI cells and AMs stimulated with a range of smooth form LPS (S-LPS, A) and rough form LPS (R-LPS, B) in medium containing 5% serum from wt or LBP deficient mice (A, B). TNF-α levels in supernatants of mock stimulated and smooth or rough form LPS (0.1 µg/ml) treated BMMs and MPI cells in serum free medium containing 0, 0.06 or 0.3 µg/ml recombinant LBP (C). TNF-α levels of alveolar macrophages from wt and CD14 deficient mice stimulated with 0.01 µg/ml R-LPS in serum-free medium in the presence or absence of 0.3 µg/ml recombinant LBP (D). TNF-α levels of BMMs (left panel) and MPI cells (right panel) from CD14 deficient mice stimulated with R-LPS or S-LPS (0.1 µg/ml, each) in medium containing 5% of serum from wt or LBP deficient mice (E)

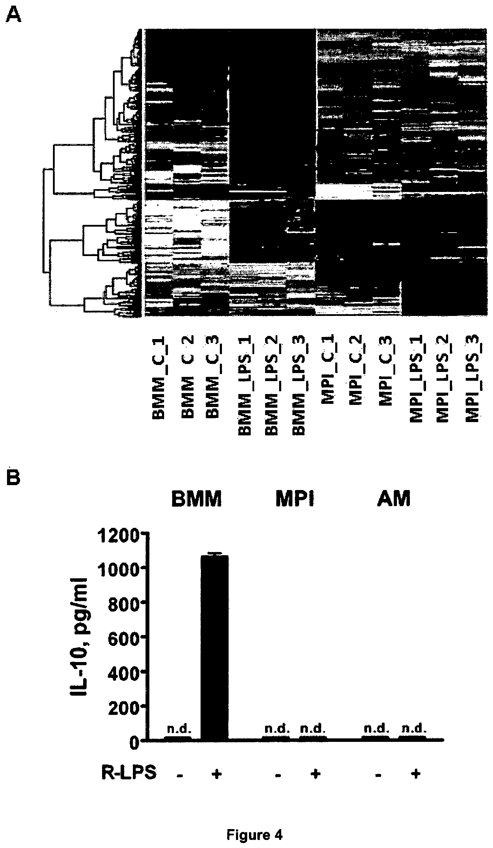

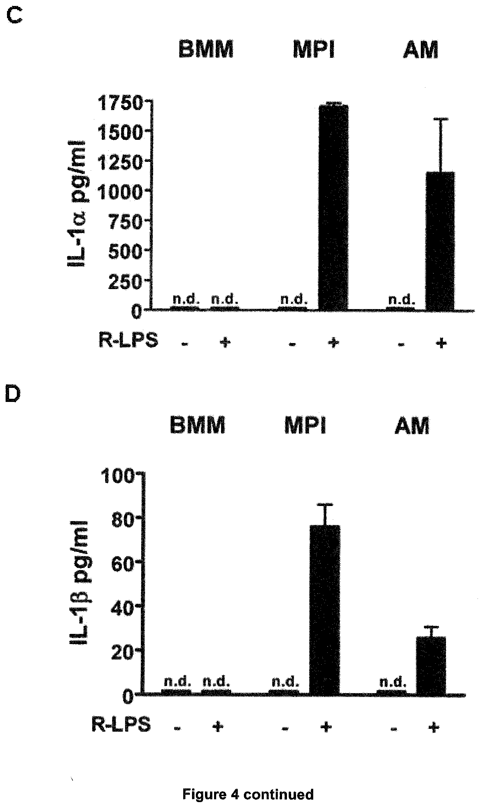

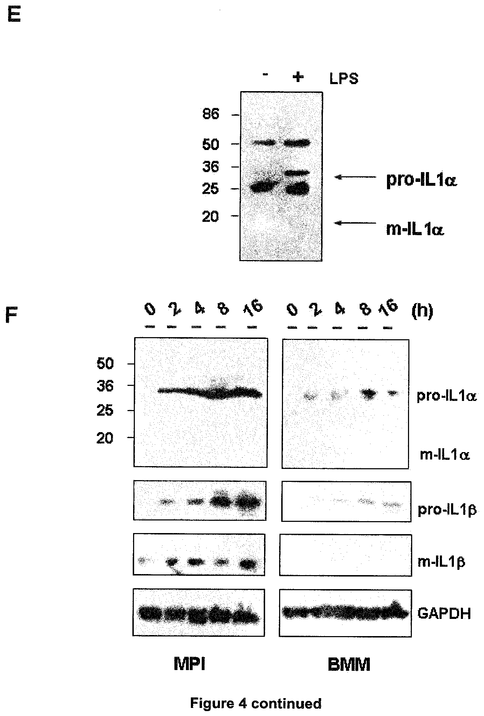

**Figure 4****:** Differences between LPS stimulated responses of MPI cells and BMMs. Heatmap of genes differentially induced by LPS in BMMs and MPI cells (A) Induction of IL-10 (B), IL-1α (C) and IL-1β (D) in supernatants of BMMs, MPI cells and alveolar macrophages stimulated with 0.1 µg/ml R-LPS. Measurement of pro- and mlL-1α in supernatants of mock treated and 0.1 µg/ml R-LPS stimulated MPI cells with immunoprecipitation followed by immunoblotting (E). Measurement of pro- and mature IL-1α and IL-1β in lysates of MPI cells and BMMs at various time-points after stimulation with 0.1 µg/ml R-LPS (F).

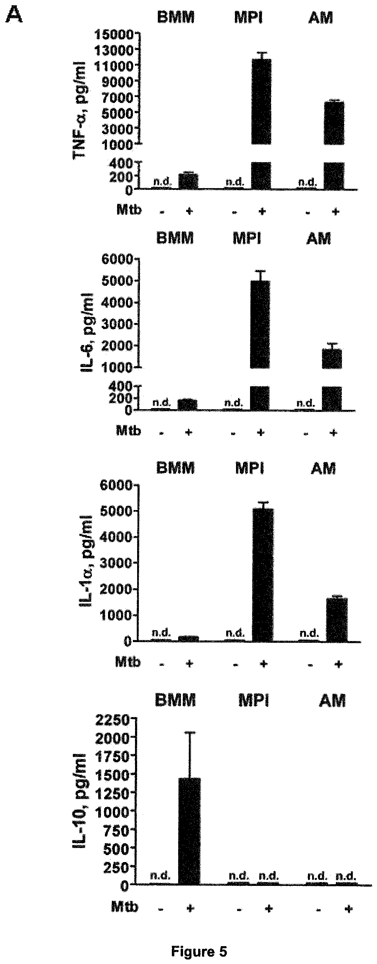

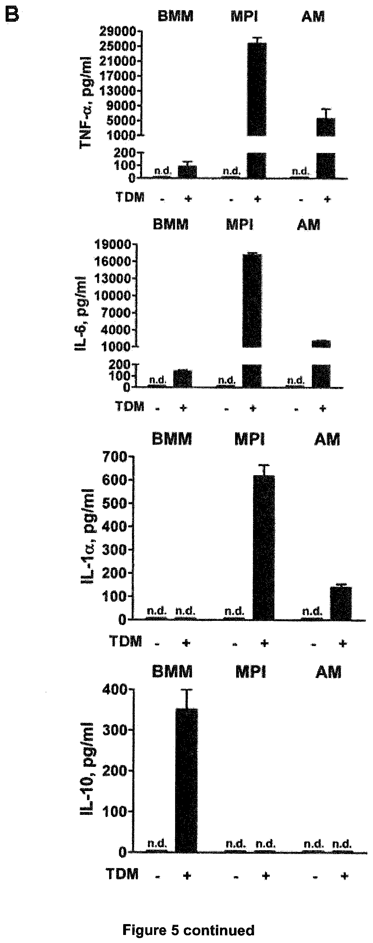

**Figure 5****:** Cytokine responses to heat killed *Mycobacterium tuberculosis* and trehalose dimycolate in MPI cells and alveolar macrophages. Measurement of TNF-α, IL-6, IL-1α and IL-10 in supernatants of BMMs, MPI cells and alveolar macrophages stimulated with heat killed *Mycobacterium tuberculosis* at 20 bacterial particles/cell (A) or with 25 ng/ml trehalose dimycolate (B).

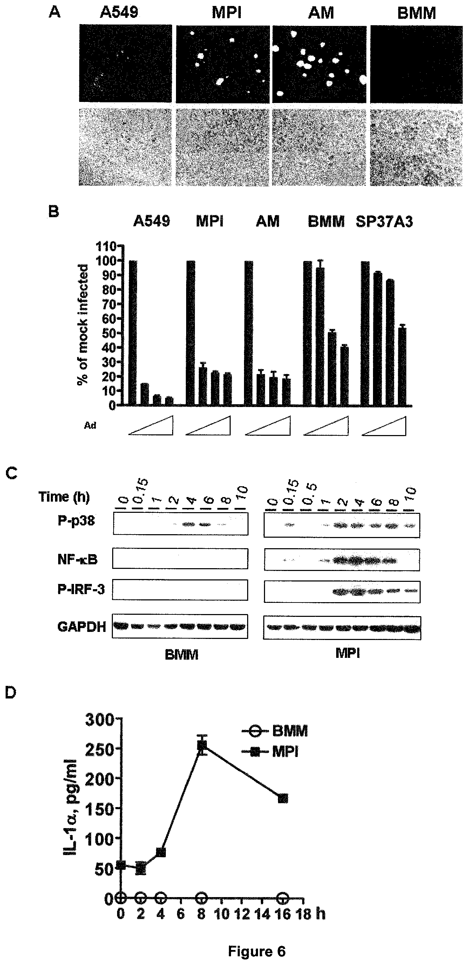

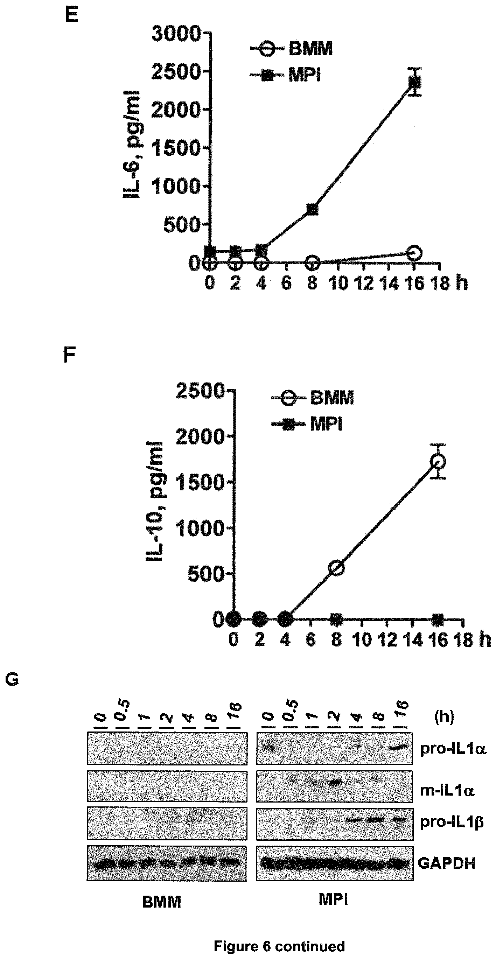

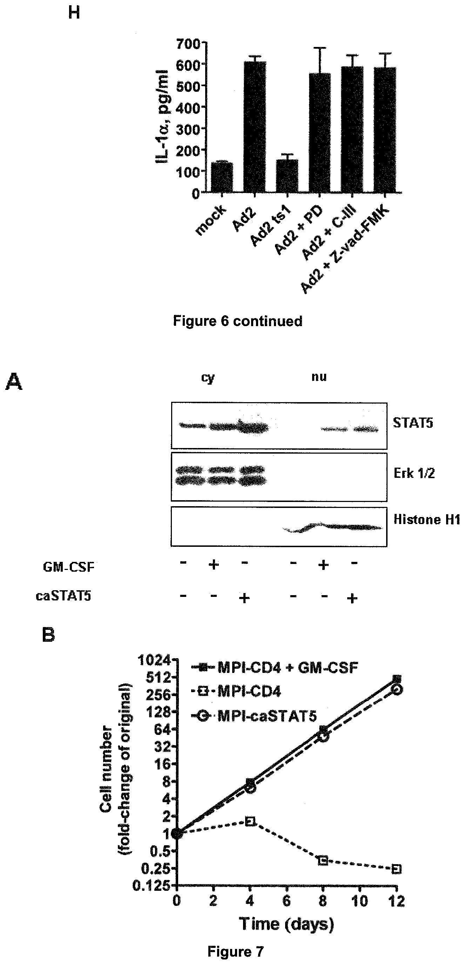

**Figure 6****:** Highly efficient virus entry and robust pro-inflammatory responses in adenovirus infected MPI cells. A549, MPI cells, alveolar macrophages and BMMs have been infected with ad5GFP (100 viral particles/cell) and GFP expressing cells were visualized 16 hrs after virus infection (A). Sarcin assay in A549, MPI, SP37A3 cells, AMs and BMMs using 0, 100, 1000 and 10000 Ad2 viral particles/cell (B). Activation of p38, NF-κb p65 and IRF-3 in BMMs and MPI cells at various time-points after infection with 2000 Ad5 GFP viral particle/cell (C). Measurement of IL-1α (D), IL-6 (E), and IL-10 (F) in supernatants of BMMs and MPI cells stimulated with Ad5GFP at 2000 viral particle/cell at different time-points after virus infection. Measurement of pro- and mature IL-1α and IL-1β in lysates of MPI cells and BMMs at the indicated time-points after stimulation with 2000 viral particles/cell Ad5 GFP (G). Induction of IL-1α in in supernatants of MPI cells stimulated with 2000 viral particles/cell Ad2, Ad2ts1 and Ad2 in the presence of the Calpain inhibitor PD 150606 (PD), Calpain inhibitor III (C-III) and Z-vad-FMK 16 hrs after infection (H).

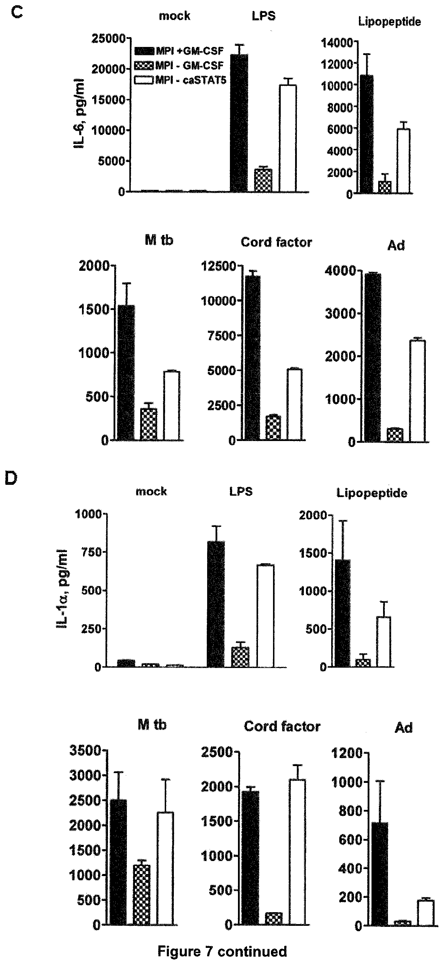

**Figure 7****:** STAT5 plays an important role in the self-renewing capacity and innate reactivity of MPI cells. Detection of STAT5 in cytoplasmic (cy) and nuclear (nu) extracts of GM-CSF incubated and starved MPI cells and in MPI cells expressing caSTAT5 cultured without GM-CSF (A). Growth curve of GM-CSF incubated and starved MPI cells and caSTAT5 expressing MPI cells in the absence of GM-CSF (B). IL-6 (C) and IL-1α (D) responses of GM-CSF incubated or starved MPI cells and caSTAT5 MPI cells to 0.1 µg/ml R-LPS, 0.1 µg/ml lipopeptide FSL, 10 bacterial particles/cell heat killed *Mycobacterium tuberculosis* (Mtb), 25 ng/ml trehalose dimycolate (cord factor) and 2000 viral particles/cell Ad5 GFP. IL-6 levels of MPI cells and caSTAT5 MPI cells stimulated with a range of rough form LPS in medium containing 5% serum from wt or LBP deficient mice (E).
**Figure 8****:**
   A: MPI cells are not tumorigenic in vivo. Groups of T, B and NK cell deficient Rag/C mice (4/group) were injected with Lewis Lung carcinoma cells (3LL) intraperitoneally or with MPI-2 cells (MPI) subcutaneously or intraperitoneally and the animals were checked for survival and the appearance of tumors. The number of living, tumour-free animals is shown.
   B: Adoptive transfer of MPI cells enables LPS responses in TLR4 deficient ice. Groups of TLR4 deficient mice were left non-injected or injected with MPI cells made from wild type or TLR4 deficient mice. Wild type and various groups of TLR4 deficient mice (3 mice/group) were challenged 16 hrs later with 10 µg LPS intraperitoneally and the levels of TNF were measured in plasma samples 1 and 2 hrs after LPS injection.
   C: LPS treatment and GM-CSF deprivation arrests replicating MPI cells in the G1 phase of cell cycle. MPI cells transduced with a retrovirus coding for CD8 and a reporter protein emitting (in PE-A channel) in G1 phase only were analysed when grown with GM-CSF (upper 2 panels), 16 hrs after treatment with 0.1 µg/ml R-LPS (upper right panel) and 2 days after GM-CSF deprivation (lower right panel). Control non-transduced GM-CSF cultured MPI cells are shown in lower left panel.

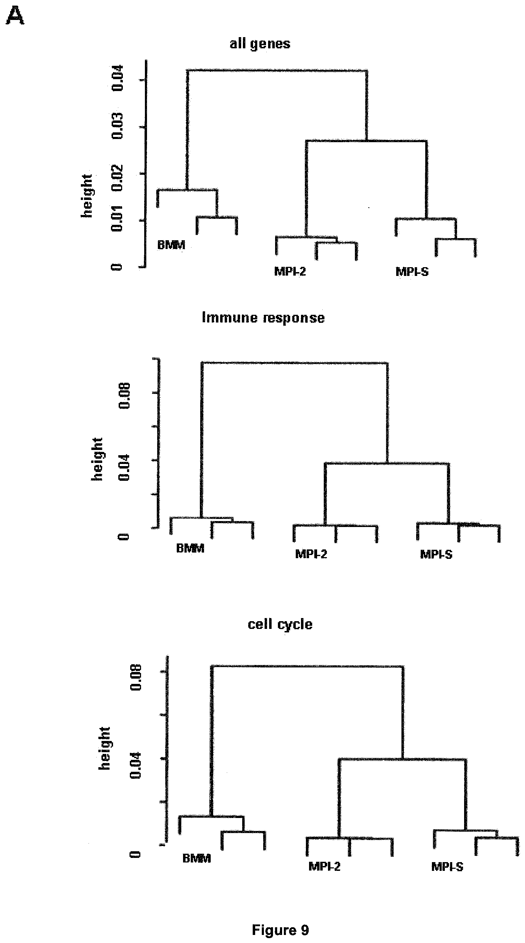

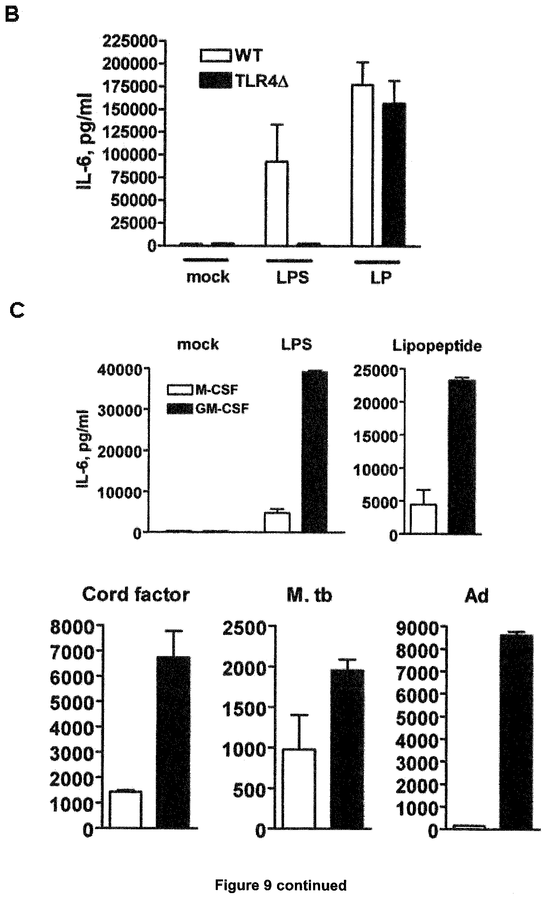

**Figure 9****:** Gene expression profiles and innate responses in MPI cells. Cluster analysis for all genes, immune response genes and cell cycle genes in BMMs, MPI-2 cells from fetal liver (MPI) and MPI cells from spleen (MPI-S) using newly synthesized RNAs obtained with 4tU labelling and microarrays. (A) IL-6 levels of wt (MPI-wt), TLR4 deficient (MPI-ΔTLR4) and mouse TLR4 deficient, human TLR4 expressing (MPI-hTLR4) MPI cells in response to 0.1 µg/ml R-LPS and 0.5 mM nickel cloride (B). IL-6 responses of MPI cells grown with either GM-CSF or M-CSF to 0.1 µg/ml R-LPS, 0.1 µg/ml lipopeptide FSL, 10 bacterial particle/cell heat killed *Mycobacterium tuberculosis* (Mtb), 25 ng/ml trehalose dimycolate (cord factor) and 2000 viral particles/cell Ad5 GFP.

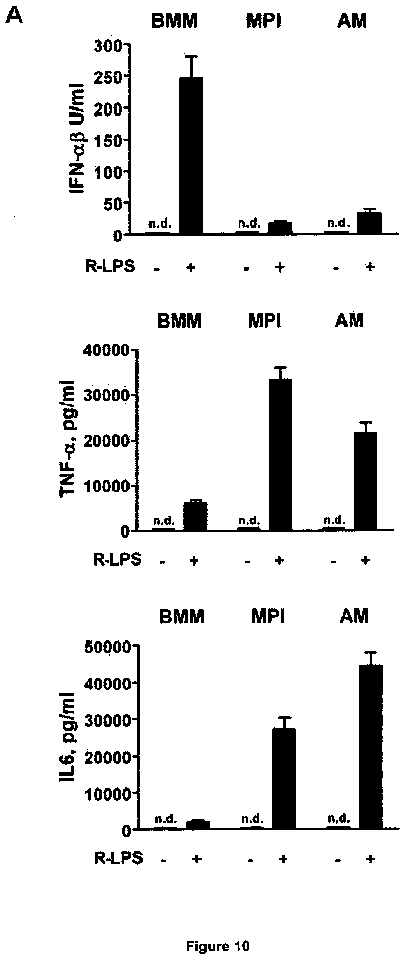

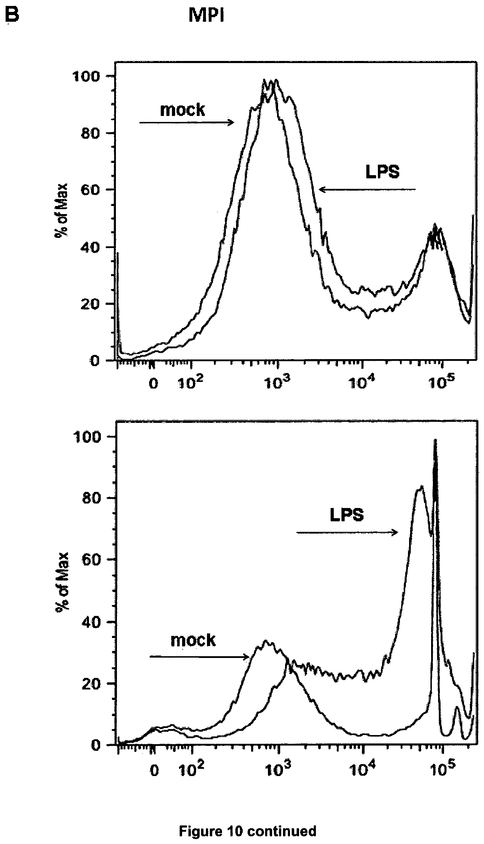

**Figure 10****:** Differences between LPS stimulated responses of MPI cells and BMMs. Induction of IFN-αβ, TNF-α and IL-6 in supernatants of BMMs and MPI cells stimulated with 0.1 µg/ml R-LPS (A). Propidium iodine staining of mock and 0.1 µg/ml R-LPS treated MPI cells and BMMs 16 hrs after stimulation (B).

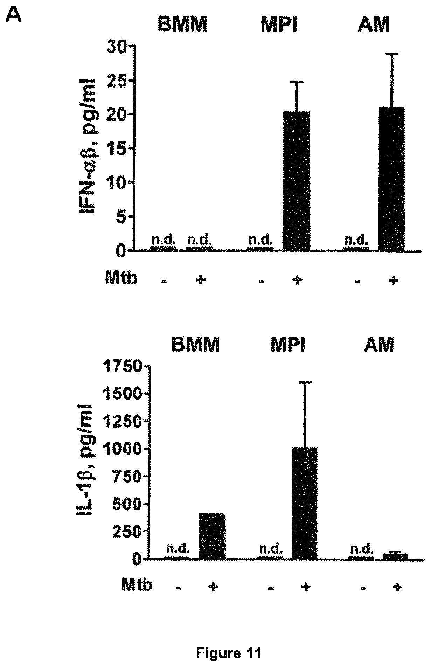

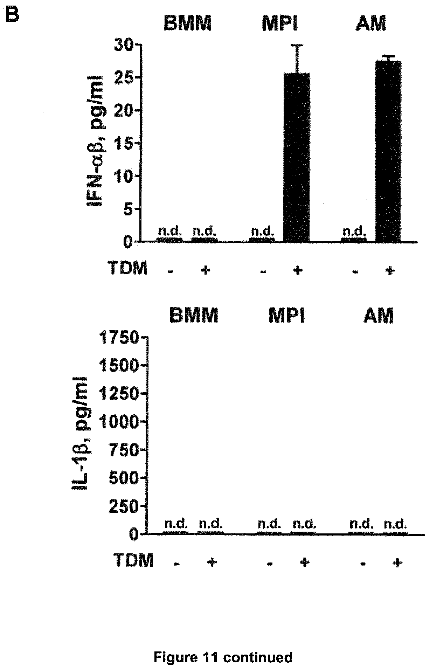

**Figure 11****:** Cytokine responses to heat killed *Mycobacterium tuberculosis* and trehalose dimycolate in MPI cells and alveolar macrophages. Measurement of IFN-αβ and IL-1β in supernatants of BMMs, MPI cells and alveolar macrophages stimulated with heat killed 20 bacterial particles/cell *Mycobacterium tuberculosis* (A) or 25 ng/ml trehalose dimycolate (B).

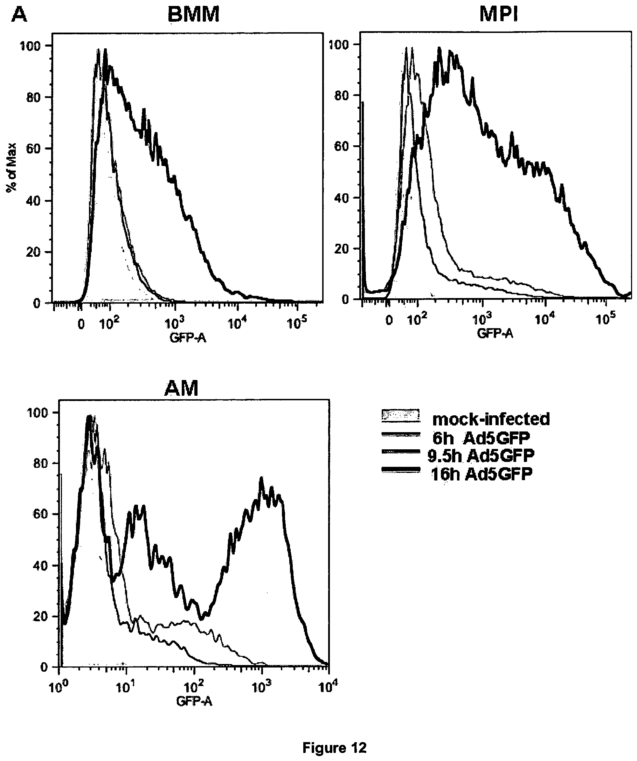

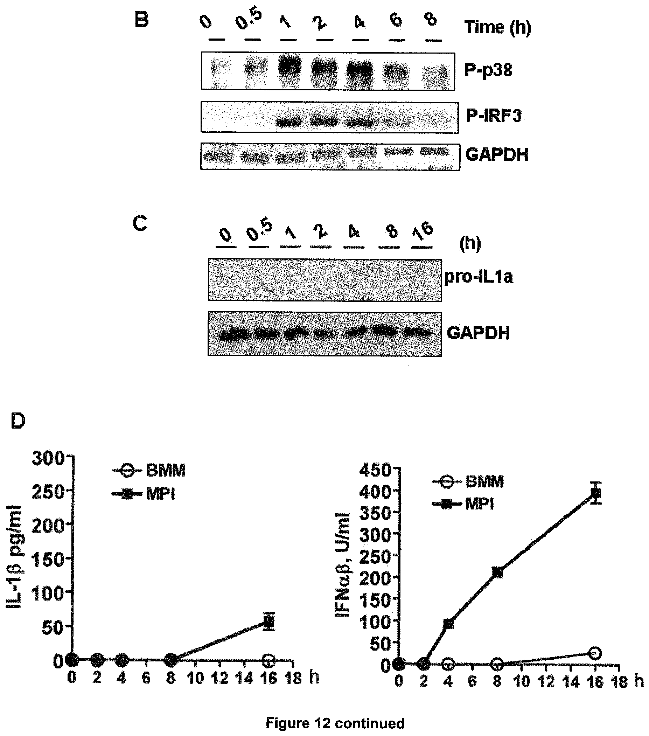

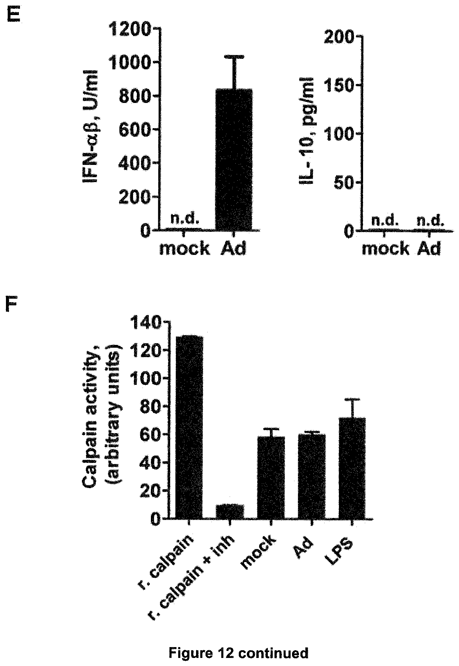

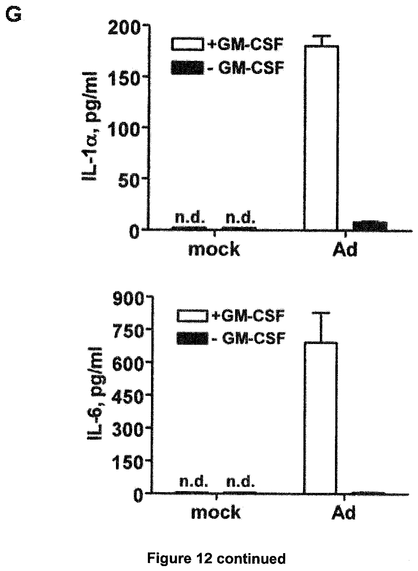

**Figure 12****:** High adenovirus infectibility and strong pro-inflammatory responses in adenovirus infected MPI cells and alveolar macrophages. BMMs, MPI cells and alveolar macrophages have been infected with 2000 viral particles/cell Ad5GFP and GFP expressing cells were measured 6, 9.5 and 16 hrs after virus infection (A). Activation of p38, and IRF-3 in alveolar macrophages at the indicated time-points after infection with 2000 Ad5 GFP viral particles/cell (B). Measurement of pro-IL-1α and in lysates of BMMs at the indicated time-points after stimulation with 2000 viral particles/cell Ad5 GFP. Long exposure of the same blot shown in Figure 6G (C). Induction of IL-1β and IFN-αβ in supernatants of BMMs and MPI cells stimulated with Ad5GFP at 2000 viral particles/cell at the indicated time-points after virus infection (D). Measurement of IFN-αβ□ and IL-10 in supernatants of alveolar macrophages stimulated with 2000 viral particles/cell Ad5GFP 16 hrs after infection (E). Calpain activity in MPI cells 2 hrs after mock treatment, infection with 2000 viral particle/cell Ad5GFP and stimulation with 0.1 µg/ml R-LPS (F). Induction of IL-1α and IL-6 in supernatants of alveolar macrophages in the presence or absence of GM-CSF in response to 2000 viral particles/cell Ad5GFP (G).

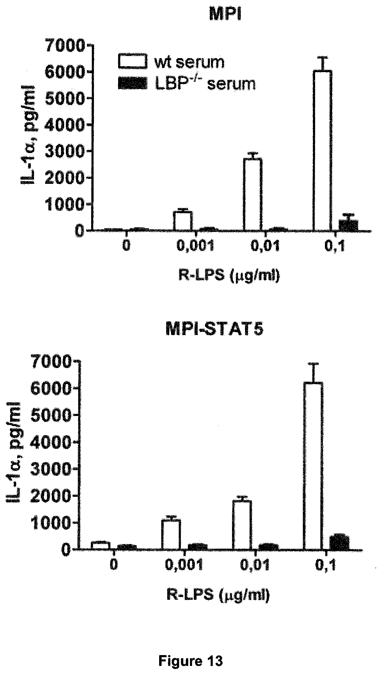

**Figure 13****:** STAT5 plays an important role in the innate reactivity of MPI cells. IL-1α levels of MPI cells and caSTAT5 MPI cells stimulated with a range of rough form LPS in medium containing 5% serum from wt or LBP deficient mice.

The examples illustrate the invention:

### Example 1: Methods and Materials

### Mouse strains

Wt C57BL/6, C57BL/10 and 129Sv mice, as well as TLR4 deficient C57BL/10ScN and T<B and NK cell deficient Balb/c-RAG/C and human TLR4 transgenic C57BL/10ScN mice were bred under SPF conditions at the MPI. All of the experimental procedures were in accordance with institutional, state and federal guidelines on animal welfare.

### Generation and culture of MPI cells

Single cell suspensions (0.5x10⁶ cells/ml) from fetal livers of 15 to 19 day-old mouse embryos or of livers of new-born mice (up to 2 weeks of age) were washed in PBS and resuspended in RPMI 1640 containing 10% fetal calf serum and supplemented with 20 ng/ml recombinant murine GM-CSF (also referred to herein as MPI medium). Rapidly proliferating cells were subcultured by splitting them 1:5 in MPI medium using cells detached with PBS/1.5 mM EDTA and floating cells in the culture. Cells proliferated more slowly after 2 weeks of culture. GM-CSF was replenished weekly and subcultures in MPI medium were made when total (attached and floating) cell density reached approximately 0.5 x 10⁶ cell/ml. After 6 to 8 weeks in culture, cells proliferated at a stable rate and stocks were cryopreserved (MPI cells). Further subcultures could be done weekly for at least 50 passages without any morphologic and functional change.

Cultures were also initiated from total cell suspensions from spleens of mice 1 to 28 days of age in MPI medium. After 1 week cells were further passaged in MPI medium containing 25% cell-free conditioned (7 days) medium from previously prepared MPI cells. The resulting constantly proliferating spleen MPI cells (MPI-S) were cryopreserved after 8 to 12 weeks in culture. Further subcultures of MPI-S cells were possible for at least 30 weekly passages without experiencing morphologic or functional changes.

### Cell culture, bacteria, viruses, sarcin assay, stimulating ligands, and inhibitors

BMMs (Bone Marrow derived Macrophages) and GM-CSF induced BMDCs were generated as described (Fejer et al., 2008). The purity of BMMs was higher than 98%, of BMDCs approximately 80%. SP37A3 cells were originally grown in RPMI in the presence of GM-CSF and M-CSF as described (Bros et al., 2007). Cells growing in media with 20 pg/ml GM-CSF but without M-CSF were selected from the original cells and used further in the studies shown here.

Alveolar macrophages were obtained with bronchial lavage using 1 ml of PBS without Ca²⁺/Mg²⁺ four times through the trachea and cultured in RPMI containing 20 pg/ml GM-CSF. L-929 cells were grown in DMEM with 10% FCS.

Heat-killed Propionibacterium acnes was obtained as described (Kalis et al., 2005). Human Adenovirus of species C Ad serotype 2, Ad2ts1 and Ad5-GFP (early gene expression defective Ad) were grown and purified twice on caesium chloride density gradients as described previously (Fejer et al., 2005). The ratio of infectious/total viral particles was determined on susceptible cells and was typically 1:20-50. If not otherwise stated Ad2 ts1 used for the experiments was grown at non-permissive temperature 38.5°C.

Sarcin assay, measuring Ad endosomal escape was done as described previously (Schreiner et al., 2012). Ultrapure S-form LPS from S. *abortus equi*, and R-form LPS from S. *minnesota* mutant R 595 were extracted from parent bacteria and further purified as described (Huber et al., 2006). Lipopeptide FSL-1, CpG ODN 1668 and polyinosinic-polycytidylic acid (polyl:C) were purchased from Invivogen. Recombinant murine LBP was purchased from Biometec. Non-endotoxin ligands were substantially LPS-free (less than or equal to 1 pg LPS/1011 viral particles) as determined by the Limulus amebocyte lysate test from Pyroquant. Calpain inhibitors PD 150606 and Calpain inhibitor III were from Sigma and the pan-caspase inhibitor Z-VAD-FMK from Biovision.

*Detection of secreted cytokines*, *surface antigens*, *intracellular proteins and enzymatic activity* The following cytokines were detected with ELISA using commercially available antibody pairs or kits: mouse IL-6 (BD Bioscience), IL-1α (eBioscience), IL-1β (eBioscience) and IL-10 (eBioscience), human IL-6 (R&D Systems), IL-1α (eBioLegend) and IL-10 (BD Bioscience). IFN-αβ and TNF-α activities were measured with bioassays as described (Fejer et al., 2005). Surface markers were detected with FACS using the following antibodies: I-Ab (AF6-120.1), Gr-1 (RB6-8C5) and CD11b (M1/70) from BD Pharmingen, F4/80 (BM8) and CD11c (N418) from eBioscience, CD32/16 (93) and CD14 (Sa14-2) from BioLegend, MARCO (ED31) from AbD Serotec. Intracellular proteins were detected on immunoblots with antibodies from Pharmingen BD (IL-1α and IL-1β) and Cell Signalling (phosphorylated isophorm of p38 MAPK). Calpain activity was measured from cell lysates with a commercial kit from Biovision.

### Preparation of RNA samples and global gene expression profiling

Total cellular RNA was prepared with Trizol (Invitrogen). Newly synthesized RNA was obtained with 4tU labelling of cells at 250 µM in culture medium for 60 min and was affinity purified as described previously (Dolken et al., 2008). RNA samples were amplified and labeled using the Affymetrix One-Cycle Target Labeling Kit and Biotinylated cRNA was hybridized to Affymetrix MG 430 2.0 arrays according to the manufacturer's recommendations.

### Data analysis and statistics

Data was analyzed using Prism GraphPad 4.0 software. Data in all figures are presented as mean, error bars show SEM.

### Example 2: MPI lines are self-renewing, GM-CSF-dependent non transformed cells.

To study the reactivity of tissue macrophages to microbial stimuli, a method was developed for the generation of murine self-renewing, GM-CSF-dependent macrophages, designated MPI cells, from different organs as described above in "Materials and Methods". So far MPI cell lines have been successfully established from wild type and knockout mice of several genetic backgrounds (C57BI/6, C57BI/10, 129Sv/Pas, BALB/c). If not otherwise stated, data are report herein that were obtained with MPI cells generated from fetal liver of wild type C57BL/6 mice.

In the presence of GM-CSF, MPI cells grow exponentially, are predominantly adherent and approximately 0.1 % of the cells are multinuclear giant cells (Fig. 8A and Fig.1B). The majority of the cells exhibits a characteristic round shape similar to that of alveolar macrophages and different from the multi-angular shape that is typical for bone marrow-derived macrophages (BMMs) or dendritic cells (DCs) (Fig.1A). Their morphology and function remained stable for at least 130 weekly passages. The replacement of GM-CSF by M-CSF slowed down the replication rate of the cells whereby no significant change of cell morphology appeared. The replacement of GM-CSF by IL-3, another factor implicated in growth and differentiation of myeloid-cells, or its complete removal resulted in stop of cell replication because of G1 arrest and a slow reduction in the number of living cells in culture (Figs. 1B and 8B). The lack of tumorigenic transformation capability of the cells of the invention has been tested by injection of them into RAG/C mice. This experiment showed no evidence of tumor formation even in this B, T and NK cell deficient host (see Figure 2E).

### Example 3: The expression of characteristic surface antigens and the uptake of bacteria suggest the affiliation of MPI cells to macrophages

To characterize the self-renewing MPI cells, the expression of several surface antigens, characteristic for cells of myeloid lineage (Fig1C) was examined. It was found that MPI cells, like BMMs and BMDCs, are positive for CD11b, F4/80 and CD32 and negative for Gr-1. Furthermore, in contrast to negative BMMs and strongly positive BMDCs, they exhibit weak CD11c expression. Finally, like BMMs and unlike BMDCs they do not express MHC class II (Fig 1C). In agreement with these findings and contrary to BMDC, MPI cells were not able to present allergens to T cells in an *in vivo* mouse model of contact hypersensitivity (Fig.1D), confirming the lack of MHC class II expression on MPI cells.

Because phagocytosis of microbes is a characteristic function of macrophages, it was tested whether MPI cells could internalize microbial agents. Incubation of the cells with heat-killed *Propionibacterium acnes* resulted in the efficient uptake of the bacteria (Fig 1D).

### Example 4: Global gene expression profiling indicates that MPI cells are alternatively differentiated macrophages, similar to alveolar macrophages.

In order to gain a comprehensive view of characteristic genes, the expression levels of more than 20 000 genes was compared in MPI cells, BMMs and the splenic myeloid DC line SP37A3 by analyzing RNA levels using microarrays. Cluster analysis showed that two independently made MPI lines were very close to each other and significantly closer to BMMs than to the SP37A3 cells when all the genes or "immune response" genes were analyzed (Fig2A left and middle panel). However, when the analysis was carried out on "cell cycle" genes, MPI cells appeared more similar to SP37A3 cells than to BMMs reflecting the self-renewing capacity of the two former cell types (Fig2A right panel). Furthermore, in an independent set of microarrays an MPI line prepared from spleen (MPI-S) clustered close to the MPI cells prepared from fetal liver and both lines were well separated from BMMs (Fig 9A). An overlap and gene set analysis for enriched pathways revealed that main differences between MPI cells and BMMs were found mainly in "Mitotic genes" ("cell cycle", "DNA binding", "chromatin assembly", "DNA replication", etc.) and some immune response subcategories ("TLR pathway", "JNK pathway"). Differences between MPI and SP37A3 cells were in "immune response" with several subcategories (e.g. "antibacterial defence", "lipoprotein binding", "chemokine receptor binding ", "double stranded RNA binding", etc.) and in "myeloid differentiation". Overall, this analysis indicated that MPI cells represent macrophages, rather than dendritic cells.

Among myeloid cell markers high levels of *MARCO* mRNA and low levels of CD11c mRNA were found in MPI cells, while neither MARCO nor CD11c were expressed in BMMs. In accordance, MARCO and CD11c were expressed at the protein level only in MPI cells but not in BMMs (Fig. 2B and 1C). On the other hand, the expression of the LPS-binding protein CD14 on both the mRNA and protein (Fig 2B) levels were higher in BMMs then in MPI cells. Of note, the presence of MARCO, CD11c and low levels of CD14 is characteristic for alveolar macrophages (Landsman and Jung, 2007; Palecanda et al., 1999).

### Example 5: Selected TLR ligands induce differential cytokine responses in MPI cells and BMMs.

Recognition of microbial agents and subsequent production of cytokines is a hallmark of macrophage function and proteins of the Toll-like receptor family play an important role in this function. MPI cells are potent producers of pro-inflammatory cytokines IL-6 and TNF-α, especially via TLR 2 and 4. In comparison to BMMs they produced higher levels of IL-6 and TNF-α upon stimulation with the TLR4-dependent bacterial lipopolysaccharide (LPS; TLR4 ligand) or the TLR2-dependent lipopeptide FSL-1 (Fig 2C, D). Furthermore, both MPI cells and BMMs produced comparable levels of the two cytokines also after stimulation with the TLR3 activator synthetic double stranded (ds)RNA (Fig 2C). However, in comparison to BMMs, MPI cells were much lower producers of IFN-αβ in response to LPS and dsRNA, while neither of the cell types produced IFN-αβ upon stimulation with FSL-1 (Fig 2C). The cytokine responses to the TLR9-dependent synthetic CpG DNA (CpG ODN 1668) were marginal or similarly absent in both cell types (Fig 2D). To show that MPI cells generated from knockout or transgenic mice exhibit the expected biological properties, the induction of cytokines through TLR4 was determined in MPI cells generated from fetal liver of TLR-deficient or human TLR4-expressing mice. In a control experiment, such cells responded normally to the TLR2-dependent FSL-1 (Fig. 9B and not shown). As expected, the absence of TLR4 resulted in the loss of LPS responsiveness, while the presence of human TLR4 endowed the cells with the ability to respond not only to LPS but also to nickel (Fig.2D and 9B) confirming previous results on BMMs and BMDCs (Schmidt et al., 2010).

### Example 6: Differential requirement for LPS-binding accessory proteins in activation of the LPS receptor by rough form LPS in MPI cells and alveolar macrophages and BMMs.

The finding that MPI cells are highly susceptible to LPS, the probably most potent activator of the innate immune system, lead to a closer investigation of the interaction of LPS with MPI cells. According to current consensus, the activation of the signalling receptor TLR4/MD2 depends on the LPS chemotype. While the activation with smooth form (S)-LPS requires the aid of the LPS-binding proteins LBP and CD14, the activation with rough form (R)-LPS in various mouse immune cells, including BMMs (Fig 3B,C,E), peritoneal macrophages, mast cells or B-cells occurs efficiently also in the absence of LBP and CD14 (Huber et al., 2006; Jiang et al., 2005). Surprisingly however, it was found here that the activation of MPI cells by both S- and R-LPS is highly LBP dependent (Fig 3A-C). In contrast to BMMs, substantial TNF-α or IL-6 responses to R- and S-LPS were induced in MPI cells only in the presence of LBP, whose function is to deliver LPS to the accessory CD14 on membrane of TLR4-positive cells (Fig 3A-C, Fig 7D). Furthermore, although R-form LPS induced TNF-α in CD14-deficient BMMs, no TNF-α was induced in similarly treated CD14-deficient MPI cells (Fig. 3E). Interestingly, a similar requirement for LBP and CD14 assistance in the induction of TNF-α by both S- and R-LPS was observed in alveolar macrophages (Fig 3A, B, D).

### Example 7: Similarities and differences in LPS-induced gene activation between MPI cells and BMMs

MPI cells and BMMs were stimulated with LPS and changes in global gene expression profiles were followed by microarray analysis of newly synthesized RNA labeled between 30-90 minutes of stimulation. This very early time-point enabled the study of mainly the primary components of the LPS effects. Gene set analysis revealed the enrichment of common activated pathways characteristic for innate responses (e.g. "inflammatory response", "cytokine production", "defence response", "locomotory behaviour", etc) in both BMMs and MPI cells. However, there were differences regarding the individual components of these pathways between the two cell types. For example, basal and induced levels of IL-1α and IL1-β were higher in MPI cells than in BMMs. Furthermore, CD14, Ereg, CSF-3, CXCI2 and CXCL10 were more strongly induced in MPI cells than in BMMs. On the other hand, IL-10, CXCL9, CXCL11, Mx1 and IFN-β were more strongly induced in BMMs. Differences in LPS induced gene expression are demonstrated in Fig4A.

To verify the differences in LPS-induced gene activation between MPI cells and BMMs seen on mRNA level, we determined the levels of IL-10, IFN-β, TNF-α and IL-6 secreted by LPS-stimulated cells. In accordance with the microarray analysis data, stimulated MPI cells, contrary to similarly treated BMMs, did not produce IL-10 (Fig 4B). Furthermore, contrary to BMMs, they secreted significantly less IFN-αβ, but more IL-6 and TNF-α (Fig 10A). Notably, the pattern of LPS-induced IL-10, IFN-β, TNF-α and IL-6 response in MPI cells was similar to that of AMs (Fig 4 B-D, 10A).

IL-1α and IL-1β are key pro-inflammatory cytokines but according to the current consensus, these factors are induced by LPS both only as pro-cytokines and a different, second signal is required to obtain the protease-cleaved mature, secreted protein form (Dinarello, 2009; Fettelschoss et al. 2011). The lysates of LPS-stimulated MPI cells contained substantially higher levels of pro-IL-1α and β than those of BMMs and both cytokines were produced in MPI cells more rapidly than in BMMs. Tthe cleaved mIL-1α could not be detected in any of the two cell types; however, LPS treatment induced the production of m IL-1β in MPI cells but not in BMMs (Fig 4C). Interestingly, very high amounts of unprocessed pro-IL-1α were found in supernatants of LPS-stimulated MPI cells, but nothing in those of BMMs (Fig 4D, E). By showing that there is no difference in viability between LPS- and mock-stimulated cells it was excluded that LPS was toxic and the pro-IL-1α originated from dying MPI cells (Fig. 10B). Of note, MPI cells share the capability to secrete IL-1α after LPS treatment with AMs (Fig 4D). Interestingly, also some processed IL-β was found in lysates of LPS-stimulated MPI cells, but not in those of similarly treated BMMs (Fig 4C). Moreover, LPS stimulated MPI cells and AMs, unlike BMMs, secreted low amounts of IL-1β (Fig 4F). Overall, the pattern of cytokine response to LPS documents that both MPI cells and AM exhibit a similar, strong pro-inflammatory phenotype.

### Example 8: MPI cells and AMs are extremely sensitive to secrete pro-inflammatory cytokines but fail to produce IL-10 in response to Mycobacterium tuberculosis and cord factor

The striking similarities between MPI cells and AMs raised the question how these cells react to air-born pathogens. The reactivity of the MPI cells and AMs was compared to that of BMMs after stimulation with the pulmonary pathogen *Mycobacterium tuberculosis* (Mtb), and its innate stimulatory component, trehalose dimycolate (TDM; cord factor). Comparison of the cytokine responses of the different cell types to the above stimuli revealed striking differences between MPI cells and AMs on one side and BMMs on the other as shown on Figures 5 and 11. Mtb- and cord factor stimulated MPI cells and AMs produced high levels of TNF-α, IL-6 and IL-1α, but no detectable IL-10 (Fig 5A and B). In contrast, BMMs produced only very low amounts, if any of TNF-α, IL-6 and IL-1α, but relatively high amounts of IL-10 in response to these stimuli (Fig 5A and B). Furthermore, MPI cells and AMs but not BMMs produced small but detectable amounts of INF-αβ in response to Mtb and TDM (Fig 11). In addition, BMMs and MPI cells produced moderate and AMs low levels of IL-1β in response to Mtb (Fig 11A), while TDM did not induce this cytokine in any of the macrophage types (Fig 11B).

### Example 9: MPI cells and AMs are highly permissive to adenoviral infection

Adenoviruses (Ads) are another important type of air-born pathogens. Human Ads infect immune cells efficiently both in humans and mice and virus entry elicits a vigorous innate response requiring endosomal escape (Fejer et al., 2008). However, in currently available *in vitro* systems Ad infection is inefficient and requires very high multiplicities of infection (Barlan et al., 2011; Fejer et al., 2008; Nociari et al., 2007; Zhu et al., 2008).

The permissiveness of MPI cells to infection with a GFP expressing species C defective Ad (Ad GFP) was assayed. GFP expression was compared in the highly Ad sensitive human lung carcinoma cell A549, in MP cells, AMs and BMMs 16 hrs after infection with Ad GFP at a low multiplicity of infection (moi) of 5 pfu/cell. Hardly any GFP-expressing cells were observed in BMMs, but almost 100% in A549 cells with fluorescent microscopy. However, many GFP-expressing cells were detected in MPI cells and AMs, comparable to those of A549 cells (approximately 50%). GFP expression was also compared in the different cell types after transduction with Ad GFP at a 10 times higher moi using FACS analysis. In this case, GFP-expressing cells could be detected in MPI cells and AMs but not in BMMs as early as 6 hrs after transduction. Weakly GFP-positive cells were detected first 16 hrs after transduction in BMMs, however, by that time more strongly positive (10-100 times) MPI cells and AMs were found.

Since facilitated virus entry could be involved in the observed very high transduction rate of MPI cells, this step was tested by comparing the efficiency of Ad endosomal escape in MPI cells, AMs, BMMs, SP37A3 cells and A549 cells. Sarcin assay revealed that Ad endosomal escape is very efficient in MPI cells and AMs and comparable to what is experienced in A549 cells 2 hrs after virus infection at various multiplicities of infection. Endosomal escape was much less efficient in BMMs and SP37A3 cells showing a more than 100-fold difference regarding viral amounts eliciting the same level of sarcin sensitivity (Fig. 6B).

### Example 10: Fast, strong and unique Ad induced innate cytokine patterns in MPI cells and AMs compared to BMMs

Adenovirus endosomal escape is crucial to elicit innate responses and it was found that this step of viral entry is significantly facilitated in MPI cells and AMs relative to BMMs (Fig. 6B). In accordance with this finding, strong activation of p38 MAP kinase and the transcription factors NF κB p65 and IRF3 was observed in MPI cells 2 hrs after virus infection that lasted for 4-6 hrs (Fig. 6C). While fast activation of p38 and IRF3 was also observed in infected AMs (Fig 12B), only delayed (4 hrs after infection) and weak activation of the p38 MAP kinase and no activation of NFκB and IRF3 was seen in Ad-treated BMMs (Fig. 6C). In agreement, substantial induction of IL-6 and IFN-αβ□ was observed only in MPI cells and AMs, but not in BMMs (Fig. 6E and 12D,E). Contrary to these pro-inflammatory cytokines, the anti-inflammatory IL-10 was readily inducible in BMMs but undetectable in MPI cells and AMs in response to Ad infection (Fig. 6F and 12E). Interestingly, Ad infected MPI cells and AMs, but not BMMs also secreted IL-1α. It was also checked whether the production of pro- or mature IL-1α was induced and it was found that Ad infection triggered the cleavage of pro IL-1α starting 2 hrs after infection and the secretion of mature IL-1α was also observed in cell free supernatants of the virus infected MPI cells. A significant IL-1β induction in lysates and supernatants of MPI cells but not in BMMs in response to Ad infection was also found (Fig 6G and 12D). In contrast to the early induction of IL-1α however, this appeared with a rather slow kinetics (4-16 hrs after virus infection).

Since the secretion of IL-1α in response to Ad has not been reported previously, the requirements for its production were analysed. It was found that the mutant adenovirus ts1, incapable of endosomal escape did not induce IL-1α in MPI cells. Similarly to previous findings in mDCs and BMMs (Fejer et al., 2008), the production of IL-6, TNF and IFN-αβ was also absent in Adts1-infected MPI cells (not shown).

Calpain activity has been previously shown to play a crucial role in the processing of IL-1α (Dinarello, 2009). Interestingly however, adenovirus induced IL-1α production was not sensitive to pharmacological blockade of calpain or caspases (Fig 6H). Furthermore, calpain activity was not increased in MPI cells after Ad infection (Fig 12F).

### Example 11 : GM-CSF-induced STAT5 confers self-renewing capacity to MPI cells and maintains their innate reactivity.

Engagement of the GM-CSF receptor leads to the induction of several pathways, including the activation of the JAK kinase family, the Shc-Ras-MAPK and phosphatidylinositol 3-kinase (PI3K) pathways and the induction of c-myc, pim-1, *cis*, PU.1 and STAT5 (Hercus et al., 2009; Shibata et al., 2001). In order to evaluate the contribution of this latter factor to the phenotype of MPI cells, they were transduced with a constitutively active form of STAT5 (caSTAT5) (Burchill et al., 2003). It was found that GM-CSF treated, proliferating MPI cells express activated STAT5, as evidenced by the presence of this protein in the nucleus (Fig 7A). Removal of GM-CSF from MPI cell cultures for 3 days resulted in the significant decrease of nuclear STAT5 levels (Fig 7A) and also to proliferation arrest (Fig. 1 and 8). Contrary to GM-CSF deprived MPI cells, however, caSTAT5 expressing MPI cells possessed this factor in their nuclei and proliferated normally in the absence of GM-CSF (Fig7A and B). The reactivity of caSTAT5 expressing MPI cells was also compared to those of cells cultured in the presence of GM-CSF or starved for this factor for 2 days. It was found that in response to various innate stimuli the latter treatment significantly diminished the production of IL-6 and IL-1α MPI cells and caSTAT5 expressing MPI cells reacted significantly better than GM-CSF starved MPI cells (Fig 7C and D). Furthermore, it was found that the need for LBP in the sensing of R-LPS is also preserved in caSTAT5-MPI cells (Fig7E and 13).

### Example 12: In vivo functionality of MPI cells.

To test if MPI cells are functional *in vivo* upon injection into animals. TLR4 deficient mice were injected with 2x10⁶ MPI cells (MPI-2=wt or TLR4 deficient MPI cells) intravenously. The MPI cells employed were either wild-type cells or cells obtained from TLR4 deficient mice. 16 hrs later mice were injected with 10µg LPS intraperitoneally and TNF levels were measured in blood samples 1 hour and 2 hours after LPS challenge.

The data provided in Figure 8B show that MPI cells are indeed functional *in vivo* upon injection into animals, as LPS induced TNF production could be detected in LPS receptor deficient mice injected with wildtype MPI cells. Therefore, the cellular source of TNF in these animals were the injected MPI cells.

### References

Ajami, B., Bennett, J.L., Krieger, C., Tetzlaff, W., and Rossi, F.M. (2007). Local self-renewal can sustain CNS microglia maintenance and function throughout adult life. Nat Neurosci 10, 1538-1543.
Barlan, A.U., Griffin, T.M., McGuire, K.A., and Wiethoff, C.M. (2011). Adenovirus membrane penetration activates the NLRP3 inflammasome. J Virol 85, 146-155.
Barreda DR, Hanington PC, Belosevic M.Regulation of myeloid development and function by colony stimulating factors. Dev Comp Immunol. 2004 May 3;28(5):509-54.
Barthel SR, Johansson MW, McNamee DM, Mosher DF; Roles of integrin activation in eosinophil function and the eosinophilic inflammation of asthma; J Leukoc Biol. 2008 Jan;83(1):1-12
Biswas, S.K., and Mantovani, A. (2010). Macrophage plasticity and interaction with lymphocyte subsets: cancer as a paradigm. Nat Immunol 11, 889-896.
Bros, M., Jahrling, F., Renzing, A., Wiechmann, N., Dang, N.A., Sutter, A., Ross, R., Knop, J., Sudowe, S., and Reske-Kunz, A.B. (2007). A newly established murine immature dendritic cell line can be differentiated into a mature state, but exerts tolerogenic function upon maturation in the presence of glucocorticoid. Blood 109, 3820-3829.
Burchill, M.A., Goetz, C.A., Prlic, M., O'Neil, J.J., Harmon, I.R., Bensinger, S.J., Turka, L.A., Brennan, P., Jameson, S.C., and Farrar, M.A. (2003). Distinct effects of STAT5 activation on CD4+ and CD8+ T cell homeostasis: development of CD4+CD25+ regulatory T cells versus CD8+ memory T cells. J Immunol 171, 5853-5864.
Chorro, L., Sarde, A., Li, M., Woollard, K.J., Chambon, P., Malissen, B., Kissenpfennig, A., Barbaroux, J.B., Groves, R., and Geissmann, F. (2009). Langerhans cell (LC) proliferation mediates neonatal development, homeostasis, and inflammation-associated expansion of the epidermal LC network. J Exp Med 206, 3089-3100.
Chow, A., Brown, B.D., and Merad, M. (2011). Studying the mononuclear phagocyte system in the molecular age. Nat Rev Immunol 11, 788-798.
Denham, S., Brookes, S.M., Hutchings, G.H. and Parkhouse, R.M.E. (1996), "Granulocyte-macrophage colony stimulating factor promotes prolonged survival and the support of virulent infection by African swine fever virus of macrophages generated from porcine bone marrow and blood", Journal of General Virology 77: 2625-2630.
Descamps D, Le Gars M, Balloy V, Barbier D, Maschalidi S, Tohme M, Chignard M, Ramphal R, Manoury B, Sallenave JM; Toll-like receptor 5 (TLR5), IL-1β secretion, and asparagine endopeptidase are critical factors for alveolar macrophage phagocytosis and bacterial killing, PNAS USA. 2012 Jan 31;109(5):1619-24
Dinarello, C.A. (2009). Immunological and inflammatory functions of the interleukin-1 family. Annu Rev Immunol 27, 519-550.
Dolken, L., Ruzsics, Z., Radle, B., Friedel, C.C., Zimmer, R., Mages, J., Hoffmann, R., Dickinson, P., Forster, T., Ghazal, P., and Koszinowski, U.H. (2008). High-resolution gene expression profiling for simultaneous kinetic parameter analysis of RNA synthesis and decay. RNA 14, 1959-1972.
Eske K, Breitbach K, Köhler J, Wongprompitak P, Steinmetz I. Generation of murine bone marrow derived macrophages in a standardised serum-free cell culture system. J Immunol Methods. 2009 Mar 15;342(1-2):13-9.
Fejer, G., Drechsel, L., Liese, J., Schleicher, U., Ruzsics, Z., Imelli, N., Greber, U.F., Keck, S., Hildenbrand, B., Krug, A., et al. (2008). Key role of splenic myeloid DCs in the IFN-alphabeta response to adenoviruses in vivo. PLoS Pathog 4, e1000208.
Fejer, G., Szalay, K., Gyory, I., Fejes, M., Kusz, E., Nedieanu, S., Pali, T., Schmidt, T., Siklodi, B., Lazar, G., Jr., et al. (2005). Adenovirus infection dramatically augments lipopolysaccharide-induced TNF production and sensitizes to lethal shock. J Immunol 175, 1498-1506.
Fettelschoss, A., Kistowska, M., LeibundGut-Landmann, S., Beer, H.D., Johansen, P., Senti, G., Contassot, E., Bachmann, M.F., French, L.E., Oxenius, A., and Kundig, T.M. (2011). Inflammasome activation and IL-1beta target IL-1alpha for secretion as opposed to surface expression. Proc Natl Acad Sci U S A 108, 18055-18060.
Fogg, D.K., Sibon, C., Miled, C., Jung, S., Aucouturier, P., Littman, D.R., Cumano, A., and Geissmann, F. (2006). A clonogenic bone marrow progenitor specific for macrophages and dendritic cells. Science 311, 83-87.
Freudenberg MA, Tchaptchet S, Keck S, Fejer G, Huber M, Schütze N, Beutler B, Galanos C.; Lipopolysaccharide sensing an important factor in the innate immune response to Gram-negative bacterial infections: benefits and hazards of LPS hypersensitivity., Immunobiology. 2008;213(3-4):193-203
Geissmann, F., Gordon, S., Hume, D.A., Mowat, A.M., and Randolph, G.J. (2010a). Unravelling mononuclear phagocyte heterogeneity. Nat Rev Immunol 10, 453-460.
Geissmann, F., Manz, M.G., Jung, S., Sieweke, M.H., Merad, M., and Ley, K. (2010b). Development of monocytes, macrophages, and dendritic cells. Science 327, 656-661.
Ginhoux, F., Greter, M., Leboeuf, M., Nandi, S., See, P., Gokhan, S., Mehler, M.F., Conway, S.J., Ng, L.G., Stanley, E.R., et al. (2010). Fate mapping analysis reveals that adult microglia derive from primitive macrophages. Science 330, 841-845.
Gordon, S., and Martinez, F.O. (2010). Alternative activation of macrophages: mechanism and functions. Immunity 32, 593-604.
Gow, D.J., Sester, D.P., and Hume, D.A. (2010). CSF-1, IGF-1, and the control of postnatal growth and development. J Leukoc Biol 88, 475-481.
Heltemes-Harris LM, Willette MJ, Ramsey LB, Qiu YH, Neeley ES, Zhang N, Thomas DA, Koeuth T, Baechler EC, Kornblau SM, Farrar MA. Ebf1 or Pax5 haploinsufficiency synergizes with STAT5 activation to initiate acute lymphoblastic leukemia. J Exp Med. 2011 Jun 6;208(6):1135-49.
Henle G, Deinhardt F. The establishment of strains of human cells in tissue culture. J Immunol. 1957 Jul;79(1):54-9.
Hercus, T.R., Thomas, D., Guthridge, M.A., Ekert, P.G., King-Scott, J., Parker, M.W., and Lopez, A.F. (2009). The granulocyte-macrophage colony-stimulating factor receptor: linking its structure to cell signaling and its role in disease. Blood 114, 1289-1298.
Huang Y, et al. Gene, 2012 Apr 25, CD16 cross-linking induces increased expression of CD56 and production of IL-12 in peripheral NK cells
Huber, M., Kalis, C., Keck, S., Jiang, Z., Georgel, P., Du, X., Shamel, L., Sovath, S., Mudd, S., Beutler, B., et al. (2006). R-form LPS, the master key to the activation ofTLR4/MD-2-positive cells. Eur J Immunol 36, 701-711.
Hume, D.A., Ross, I.L., Himes, S.R., Sasmono, R.T., Wells, C.A., and Ravasi, T. (2002). The mononuclear phagocyte system revisited. J Leukoc Biol 72, 621-627.
Inaba, K., Inaba, M., Romani, N., Aya, J., Deguchi, M., Ikehara, S., Muramatsu, S. and Steinman, R.M. (1992), "Generation of Large Numbers of Dendritic Cells from Mouse Bone Marrow Cultures Supplemented with Granulocyte/Macrophage Colony-stimulating Factor", J. Exp. Med. 176: 1693-1702.
Janeway CA Jr, Travers P, Walport M, et al.; Immunobiology: The Immune System in Health and Disease. 5th edition; New York: Garland Science; 2001
Jenkins, S.J., Ruckerl, D., Cook, P.C., Jones, L.H., Finkelman, F.D., van Rooijen, N., MacDonald, A.S., and Allen, J.E. (2011). Local macrophage proliferation, rather than recruitment from the blood, is a signature of TH2 inflammation. Science 332, 1284-1288.
Jiang, Z., Georgel, P., Du, X., Shamel, L., Sovath, S., Mudd, S., Huber, M., Kalis, C., Keck, S., Galanos, C., et al. (2005). CD14 is required for MyD88-independent LPS signaling. Nat Immunol 6, 565-570.
Kalis, C., Gumenscheimer, M., Freudenberg, N., Tchaptchet, S., Fejer, G., Heit, A., Akira, S., Galanos, C., and Freudenberg, M.A. (2005). Requirement for TLR9 in the immunomodulatory activity of Propionibacterium acnes. J Immunol 174, 4295-4300.
Katsuta H, Takaoka T, Huh N. Establishment of tissue culture cell strains from normal fetal human liver and kidney. Jpn J Exp Med. 1980 Oct;50(5):329-37.
Kawai, T., and Akira, S. (2011). Toll-like receptors and their crosstalk with other innate receptors in infection and immunity. Immunity 34, 637-650.
Kono Y, Yang S, Letarte M, Roberts EA. Establishment of a human hepatocyte line derived from primary culture in a collagen gel sandwich culture system.Exp Cell Res. 1995 Dec;221(2):478-85.
Landsman, L., and Jung, S. (2007). Lung macrophages serve as obligatory intermediate between blood monocytes and alveolar macrophages. J Immunol 179, 3488-3494.
Langermans JA, Hazenbos WL, van Furth R. Antimicrobial functions of mononuclear phagocytes. J Immunol Methods. 1994 Sep 14;174(1-2):185-94.
Lin HH, Faunce DE, Stacey M, Terajewicz A, Nakamura T, Zhang-Hoover J, Kerley M, Mucenski ML, Gordon S, Stein-Streilein J., The macrophage F4/80 receptor is required for the induction of antigen-specific efferent regulatory T cells in peripheral tolerance., J Exp Med. 2005 May 16;201(10):1615-25
Locke M. et al. 2009. Human adipose-derived stem cells: isolation, characterization and applications in surgery. ANZ J Surg 79: 235-44
Lutz MB, Granucci F, Winzler C, Marconi G, Paglia P, Foti M, Assmann CU, Cairns L, Rescigno M, Ricciardi-Castagnoli P. Retroviral immortalization of phagocytic and dendritic cell clones as a tool to investigate functional heterogeneity. J Immunol Methods. 1994 Sep 14;174(1-2):269-79.
Lutz, M.B., Kukutsch, N., Ogilvie, A.L.J., Rößner, S., Koch, F., Romani, N. and Schuler, G. (1999), "An advanced culture method for generating large quantities of highly pure dendritic cells from mouse bone marrow", Journal of Immunological Methods 223: 77-92.
Merad, M., Manz, M.G., Karsunky, H., Wagers, A., Peters, W., Charo, I., Weissman, I.L., Cyster, J.G., and Engleman, E.G. (2002). Langerhans cells renew in the skin throughout life under steady-state conditions. Nat Immunol 3, 1135-1141.
Mukhopadhyay S, Varin A, Chen Y, Liu B, Tryggvason K, Gordon S.; SR-A/MARCO-mediated ligand delivery enhances intracellular TLR and NLR function, but ligand scavenging from cell surface limits TLR4 response to pathogens. Blood. 2011 Jan 27;117(4):1319-28
Nociari, M., Ocheretina, O., Schoggins, J.W., and Falck-Pedersen, E. (2007). Sensing infection by adenovirus: Toll-like receptor-independent viral DNA recognition signals activation of the interferon regulatory factor 3 master regulator. J Virol 81, 4145-4157.
Palecanda, A., Paulauskis, J., Al-Mutairi, E., Imrich, A., Qin, G., Suzuki, H., Kodama, T., Tryggvason, K., Koziel, H., and Kobzik, L. (1999). Role of the scavenger receptor MARCO in alveolar macrophage binding of unopsonized environmental particles. J Exp Med 189, 1497-1506.
Plüddemann A, Mukhopadhyay S, Gordon S 2011. Innate immunity to intracellular pathogens: macrophage receptors and responses to microbial entry. Immunological Reviews 240:11-24.
Pollard JW Basic cell culture Methods Mol Biol. 1990;5:1-12.
Rasko J.E.J, Gough N.M, Granulocyte-macrophage colony stimulating factor, The cytokine handbook (2nd ed), A Thomson (Ed.), Academic Press, London (1994), pp. 343-369
Schmidt, M., Raghavan, B., Muller, V., Vogl, T., Fejer, G., Tchaptchet, S., Keck, S., Kalis, C., Nielsen, P.J., Galanos, C., et al. (2010). Crucial role for human Toll-like receptor 4 in the development of contact allergy to nickel. Nat Immunol 11, 814-819.
Schreiner, S., Martinez, R., Groitl, P., Rayne, F., Vaillant, R., Wimmer, P., Bossis, G., Sternsdorf, T., Marcinowski, L., Ruzsics, Z., et al. (2012). Transcriptional activation of the adenoviral genome is mediated by capsid protein VI. PLoS Pathog 8, e1002549.
Schulz, C., Gomez Perdiguero, E., Chorro, L., Szabo-Rogers, H., Cagnard, N., Kierdorf, K., Prinz, M., Wu, B., Jacobsen, S.E., Pollard, J.W., et al. (2012). A lineage of myeloid cells independent of Myb and hematopoietic stem cells. Science 336, 86-90.
Seglen PO. 1979, Hepatocyte suspensions and cultures as tools in experimental carcinogenesis. J. Toxicol Environ Health, 5:551-60
Shibata, Y., Berclaz, P.Y., Chroneos, Z.C., Yoshida, M., Whitsett, J.A., and Trapnell, B.C. (2001). GM-CSF regulates alveolar macrophage differentiation and innate immunity in the lung through PU.1. Immunity 15, 557-567.
Talbot, N.C. and Paape, M.J. (1996), "Continuous culture of pig tissue-derived macrophages", Methods in Cell Science 18: 315-327.
Weischenfeldt J., Porse B.(2008) Bone marrow-derived macrophages (BMM): isolation and applications. Cold Spring Harb. Protoc. doi: 10.1101/pdb.prot5080
Welinder E, Ahsberg J, Sigvardsson M.; B-lymphocyte commitment: identifying the point of no return.; Semin Immunol. 2011 Oct;23(5):335-40
Wen L, Hanvanich M, WernerFavre C, Brouwers N, Perrin LH, Zubler RH; Limiting dilution assay for human B cells based on their activation by mutant EL4 thymoma cells: total and anti-malaria responder B cell frequencies. European journal of Immunology 1987 17(6):887-92
Zhu, J., Huang, X., and Yang, Y. (2008). A critical role for type I IFN-dependent NK cell activation in innate immune elimination of adenoviral vectors in vivo. Mol Ther 16, 1300-1307.

## Claims

1. A method for producing self-renewing, non-transformed macrophages comprising:
culturing a cell preparation obtained from an organ obtained from a mammal in culture medium to which granulocyte macrophage colony-stimulating factor (GM-CSF) has been added; thereby differentiating the cell population into self-renewing, non-transformed macrophages,
wherein the self-renewing, non-transformed macrophages are **characterised by** the expression of at least four markers selected from the group consisting of GM-CSF receptor, M-CSF receptor, CD11c, TLR2, TLR4, TLR3, F4/80, CD16, CD11b, CD14 and Marco, and are **characterised by** a lack of expression of at least 3 markers selected from the group consisting of B220, Gr1, IgE receptor, T-cell surface glycoprotein CD3 epsilon chain and B-cell receptor.

2. The method of claim 1, wherein the cells are cultured for at least about 21 days in culture medium to which granulocyte macrophage colony-stimulating factor (GM-CSF) has been added.

3. The method of claim 1 or 2, wherein the cell preparation is obtained from fetal or neonatal organs.

4. The method of any one of claims 1 to 3, wherein the cell population is obtained from lung, liver or spleen.

5. The method of any one of claims 1 to 4, wherein the mammal is selected from the group consisting of rodents, humans, dogs, felides, pigs, ruminants or primates.

6. The method of any one of claims 1 to 5, wherein the cells of the cell preparation are cultured at a starting density of about 1x10⁵ to 10x10⁵ per ml.

7. The method of any one of claims 1 to 6, further comprising introducing a constitutively active form of STAT5 into the cells obtained by the method of any one of claims 1 to 6.

8. The method of any one of claims 1 to 7, further comprising isolating a single cell obtained by the method of any one of claims 1 to 7 and clonally expanding said cell.

9. The method of any one of claims 1 to 8, wherein the self-renewing, non-transformed macrophages obtained are free or substantially free of pathogens.

10. Macrophages obtainable by the method of any one of claims 1 to 9, wherein the macrophages are self-renewing, non-transformed macrophages being **characterised by** the expression of at least four markers selected from the group consisting of GM-CSF receptor, M-CSF receptor, CD11c, TLR2, TLR4, TLR3, F4/80, CD16, CD11b, CD14 and Marco, and being **characterised by** a lack of expression of at least 3 markers selected from the group consisting of B220, Gr1, IgE receptor, T-cell surface glycoprotein CD3 epsilon chain and B-cell receptor.

11. The macrophages of claim 10 for use in medicine or medical/pharmaceutical research.

12. A method for identifying a compound having a pharmacological, differentiating, proliferative or anti-proliferative, cytotoxic or transforming effect on the macrophages obtained by the method according to any one of claims 1 to 9 or the macrophages of any claim 10 or 11, comprising:
(a) contacting the macrophages obtained by the method according to any one of claims 1 to 9 or the macrophages of claim 10 or 11 with a test compound; and
(b) determining whether the test compound has one or more of said effects on said macrophages.

## Patentansprüche

1. Verfahren zur Herstellung von sich selbst erneuernden, nicht-transformierten Makrophagen, umfassend:
Züchten einer Zellpräparation, die aus einem von einem Säuger gewonnenem Organ erhalten wurde, in Kulturmedium, dem Granulozyten-Makrophagen-Koloniestimulierender Faktor (GM-CSF) zugesetzt wurde; wodurch die Zellpopulation in sich selbst erneuernde, nicht-transformierte Makrophagen differenziert wird,
wobei die sich selbst erneuernden, nicht-transformierten Makrophagen durch die Expression von mindestens vier Markern ausgewählt aus GM-CSF-Rezeptor, M-CSF-Rezeptor, CD11c, TLR2, TLR4, TLR3, F4/80, CD16, CD11b, CD14 und Marco gekennzeichnet sind, und durch die fehlende Expression von mindestens 3 Markern ausgewählt aus B220, Gr1, IgE-Rezeptor, T-Zell-Oberflächenglycoprotein CD3 epsilon-Kette und B-Zell Rezeptor gekennzeichnet sind.

2. Verfahren nach Anspruch 1, wobei die Zellen für mindestens etwa 21 Tage in Kulturmedium gezüchtet werden, dem Granulozyten-Makrophagen-Koloniestimulierender Faktor (GM-CSF) zugesetzt wurde.

3. Verfahren nach Anspruch 1 oder 2, wobei die Zellpräparation aus fötalen oder Neugeborenen-Organen erhalten wird.

4. Verfahren nach einem der Ansprüche 1 bis 3, wobei die Zellpopulation aus Lunge, Leber oder Milz erhalten wird.

5. Verfahren nach einem der Ansprüche 1 bis 4, wobei der Säuger ausgewählt ist aus Nagern, Menschen, Hunden, Katzen, Schweinen, Wiederkäuern und Primaten.

6. Verfahren nach einem der Ansprüche 1 bis 5, wobei die Zellen der Zellpräparation mit einer Anfangsdichte von etwa 1x10⁵ bis 10x10⁵ pro ml gezüchtet werden.

7. Verfahren nach einem der Ansprüche 1 bis 6, des Weiteren umfassend das Einführen einer konstitutiv-aktiven Form von STAT5 in die Zellen, die mit dem Verfahren nach einem der Ansprüche 1 bis 6 erhalten wurden.

8. Verfahren nach einem der Ansprüche 1 bis 7, des Weiteren umfassend das Isolieren einer Einzelzelle, die mit dem Verfahren nach einem der Ansprüche 1 bis 7 erhalten wurde, und das klonale Vermehren dieser Zelle.

9. Verfahren nach einem der Ansprüche 1 bis 8, wobei die erhaltenen sich selbst erneuernden, nicht-transformierten Makrophagen frei oder im Wesentlichen frei von Pathogenen sind.

10. Makrophagen, erhältlich durch das Verfahren nach einem der Ansprüche 1 bis 9, wobei die Makrophagen sich selbst erneuernde, nicht-transformierte Makrophagen sind, die durch die Expression von mindestens vier Markern ausgewählt aus GM-CSF-Rezeptor, M-CSF-Rezeptor, CD11c, TLR2, TLR4, TLR3, F4/80, CD16, CD11b, CD14 und Marco gekennzeichnet sind, und die durch die fehlende Expression von mindestens 3 Markern ausgewählt aus B220, Gr1, IgE-Rezeptor, T-Zell-Oberflächenglycoprotein CD3 epsilon-Kette und B-Zell Rezeptor gekennzeichnet sind.

11. Makrophagen nach Anspruch 10, zur Verwendung in der Medizin oder in medizinischer/pharmazeutischer Forschung.

12. Verfahren zur Identifizierung einer Substanz, die einen pharmakologischen, differenzierenden, proliferierenden oder anti-proliferierenden, zytotoxischen oder transformierenden Effekt auf die Makrophagen, die mit dem Verfahren gemäß eines der Ansprüche 1 bis 9 erhalten wurden, oder auf die Makrophagen nach Anspruch 10 oder 11 hat, umfassend:
(a) Inkontaktbringen der Makrophagen, die mit dem Verfahren gemäß eines der Ansprüche 1 bis 9 erhalten wurden, oder der Makrophagen nach Anspruch 10 oder 11, mit einer Testsubstanz; und
(b) Bestimmen, ob die Testsubstanz einen oder mehrere dieser Effekte auf die Makrophagen hat.

## Revendications

1. Méthode de production de macrophages non transformés, autorenouvelables comprenant :
la culture d'une préparation de cellules obtenues à partir d'un organe obtenu à partir d'un mammifère dans un milieu de culture auquel un facteur de stimulation des colonies de granulocytes et de macrophages (GM-CSF) a été ajouté ; pour différencier ainsi la population cellulaire en macrophages non transformés, autorenouvelables,
dans laquelle les macrophages non transformés, autorenouvelables sont **caractérisés par** l'expression d'au moins quatre marqueurs choisis dans le groupe constitué par un récepteur du GM-CSF, un récepteur du M-CSF, CD11c, TLR2, TLR4, TLR3, F4/80, CD16, CD11b, CD14 et Marco, et sont **caractérisés par** une absence d'expression d'au moins 3 marqueurs choisis dans le groupe constitué par B220, Gr1, un récepteur d'IgE, une chaîne epsilon de la glycoprotéine de surface CD3 des cellules T et un récepteur de cellules B.

2. Méthode selon la revendication 1, dans laquelle les cellules sont cultivées pendant au moins environ 21 jours dans un milieu de culture auquel le facteur de stimulation des colonies de granulocytes et de macrophages (GM-CSF) a été ajouté.

3. Méthode selon la revendication 1 ou 2, dans laquelle la préparation de cellules est obtenue à partir d'organes foetaux ou néonataux.

4. Méthode selon l'une quelconque des revendications 1 à 3, dans laquelle la population de cellules est obtenue à partir du poumon, du foie ou de la rate.

5. Méthode selon l'une quelconque des revendications 1 à 4, dans laquelle le mammifère est choisi dans le groupe constitué par les rongeurs, les sujets humains, les chiens, les félidés, les porcs, les ruminants ou les primates.

6. Méthode selon l'une quelconque des revendications 1 à 5, dans laquelle les cellules de la préparation de cellules sont cultivées à une densité de départ d'environ 1x10⁵ à 10 x 10⁵ par ml.

7. Méthode selon l'une quelconque des revendications 1 à 6, comprenant en outre l'introduction d'une forme constitutivement active de STAT5 dans les cellules obtenues par la méthode selon l'une quelconque des revendications 1 à 6.

8. Méthode selon l'une quelconque des revendications 1 à 7, comprenant en outre l'isolation d'une cellule unique obtenue par la méthode selon l'une quelconque des revendications 1 à 7 et l'expansion par clonage de ladite cellule.

9. Méthode selon l'une quelconque des revendications 1 à 8, dans laquelle les macrophages non transformés, autorenouvelables obtenus sont exempts ou substantiellement exempts de pathogènes.

10. Macrophages pouvant être obtenus par la méthode selon l'une quelconque des revendications 1 à 9, où les macrophages sont des macrophages non transformés, autorenouvelables **caractérisés par** l'expression d'au moins quatre marqueurs choisis dans le groupe constitué par un récepteur du GM-CSF, un récepteur du M-CSF, CD11c, TLR2, TLR4, TLR3, F4/80, CD16, CD11b, CD14 et Marco, et **caractérisés par** une absence d'expression d'au moins 3 marqueurs choisis dans le groupe constitué par B220, Gr1, un récepteur d'IgE, une chaîne epsilon de la glycoprotéine de surface CD3 des cellules T et un récepteur de cellules B.

11. Macrophages selon la revendication 10 pour une utilisation en médecine ou dans la recherche médicale/pharmaceutique.

12. Méthode d'identification d'un composé ayant un effet pharmacologique, différenciateur, prolifératif ou anti-prolifératif, cytotoxique ou transformant sur les macrophages obtenus par la méthode selon l'une quelconque des revendications 1 à 9 ou les macrophages selon l'une quelconque des revendications 10 ou 11, comprenant :
(a) la mise en contact des macrophages obtenus par la méthode selon l'une quelconque des revendications 1 à 9 ou des macrophages selon la revendication 10 ou 11 avec un composé d'essai ; et
(b) le fait de déterminer si le composé d'essai exerce un ou plusieurs desdits effets sur lesdits macrophages.
